# EUROPÄISCHE PATENTSCHRIFT

(11) **EP 2 903 493 B1**
(45) Veröffentlichungstag und Bekanntmachung des Hinweises auf die Patenterteilung: **02.08.2017**
(21) Anmeldenummer: 13777274.5
(22) Anmeldetag: 15.10.2013
(51) Int. Cl.: A61B 1/00, G02B 23/24, H04N 13/02, G02B 21/00

(54) **STEREOSKOPISCHES ABBILDUNGSSYSTEM**
STEREOSCOPIC IMAGING SYSTEM
SYSTÈME DE REPRODUCTION STÉRÉOSCOPIQUE

(30) Priorität: 16.10.2012 DE 102012218863
(43) Veröffentlichungstag der Anmeldung: 12.08.2015
(73) Patentinhaber: Arnold & Richter Cine Technik GmbH & Co. Betriebs KG, 80799 München (DE)
(72) Erfinder: MILLAHN, Manuel, 80799 München (DE); KIENING, Hans, 83661 Lenggries (DE)
(74) Vertreter: Manitz Finsterwald Patentanwälte PartmbB
(86) Internationale Anmeldenummer: PCT/EP2013/071527
(87) Internationale Veröffentlichungsnummer: WO 2014/060412

(56) Entgegenhaltungen:
- EP-A2- 1 175 106
- WO-A1-2012/047962
- US-A1- 2007 019 916
- US-A1- 2009 190 209
- US-A1- 2010 013 971
- US-B1- 6 757 021
- US-B1- 7 154 527

## Beschreibung

Die vorliegende Erfindung betrifft ein stereoskopisches Abbildungssystem in Form eines Mikroskops zur Erzeugung von Paaren von stereoskopischen Halbbildern.

Ein derartiges stereoskopisches Abbildungssystem kommt beispielsweise in Form eines Operationsmikroskops bei chirurgischen Eingriffen im Bereich der klinischen Medizin zum Einsatz. Weiterhin werden stereoskopische Abbildungssysteme auch im Bereich der biologischen und medizinischen Diagnostik verwendet.

Bei herkömmlichen stereoskopischen Operationsmikroskopen erfolgt eine vergrößerte Darstellung eines Objekts mit Hilfe von Einzelobjektiven oder Objektivpaaren und einem nachgeordneten Okularpaar, durch welches eine Person das Objekt betrachten kann. Diese Operationsmikroskope weisen den Nachteil auf, dass für ein Abzweigen von Bildinformationen aus dem Strahlengang, wie es beispielsweise für eine parallele Beobachtung durch eine zweite Person oder zu Dokumentationszwecken erforderlich ist, die für die Beobachtung durch den Hauptbenutzer zur Verfügung stehende Lichtintensität reduziert wird.

Aus dem Dokument EP 1 175 106 A2 ist ein stereoskopisches Abbildungssystem mit den Merkmalen des Oberbegriffs des Anspruchs 1 bekannt.

Die Dokumente US 6 757 021 B1 und US 2010/0013971 A1 offenbaren Videokameras, bei denen das Auslesen eines Bildsensors mit dem Verschlusswinkel bzw. der Rotationsfrequenz einer rotierenden Verschlussblende koordiniert wird.

Die Dokumente WO 2012/047962 A1, US 7154527 B1, US 2007/0019916 A1 und US 2009/0190209 A1 offenbaren weitere stereoskopische Abbildungssysteme.

Es ist die Aufgabe der vorliegenden Erfindung, ein stereoskopisches Abbildungssystem zu schaffen, welches eine Vervielfältigung der Bildinformation bei gleichbleibender Qualität und insbesondere ein Erfassen von Paaren von stereoskopischen Halbbildern unterschiedlicher Spektralbereiche ermöglicht.

Die Lösung der Aufgabe erfolgt durch ein stereoskopisches Abbildungssystem mit den Merkmalen des Anspruchs 1.

Dieser Aufbau des Abbildungssystems ermöglicht es, unter Verwendung einer einzigen gemeinsamen Empfangsoptik abwechselnd zwei Bilder aufzuzeichnen, die einer Links-Perspektive bzw. einer Rechts-Perspektive entsprechen. Hierfür bildet die Optik ein in einem Objektraum des Abbildungssystems befindliches Objekt auf einen Bildsensor ab, wobei eine Doppelaperturblende zwei zueinander versetzte Teilstrahlengänge begrenzt. Mit Hilfe einer rotierenden Verschlussblende wird abwechselnd einer der beiden Teilstrahlengänge, die der jeweiligen stereoskopischen Perspektive entsprechen, freigegeben, so dass mittels des Bildsensors ein jeweiliges Halbbild erfasst werden kann, d.h. der Bildsensor erzeugt einen jeweiligen Satz von Bildpunktsignalen. Da jedes dieser abwechselnd erfassten Halbbilder einem der beiden Teilstrahlengänge entspricht, können die stereoskopischen Halbbilder auch als stereoskopische Teilbilder bezeichnet werden. Ein jeweiliges Paar von Halbbildern ermöglicht aufgrund der zugrunde liegenden Links-Perspektive bzw. Rechts-Perspektive eine stereoskopische Wahrnehmung.

Mit Hilfe der Synchronisations- und Steuereinrichtung können die Drehzahl der Verschlussblende und die durch den elektronischen Shutter einstellbare Ladungsintegrationszeit zueinander synchronisiert werden. Unter der genannten Ladungsintegrationszeit werden der Startzeitpunkt und die Dauer oder der Endzeitpunkt der Ladungsintegration in den lichtempfindlichen Pixeln (Bildpunktelementen) des Bildsensors verstanden. Typischerweise umfasst der Bildsensor eine zweidimensionale Matrix von Pixeln, in denen in Abhängigkeit von einer Belichtung (z.B. Intensität, Dauer und Wellenlänge der Strahlungsbeaufschlagung) elektrische Ladung erzeugt und ein entsprechendes elektrisches Signal erzeugt wird (z.B. Spannungssignal). Die Bildfrequenz oder Bildrate einer vom Bildsensor erfassten Bildserie ergibt sich aus der genannten Ladungsintegrationszeit, z.B. aus der Differenz der Startzeitpunkte von zwei aufeinanderfolgend aufgenommenen Bildern.

Die Drehzahl der Verschlussblende kann insbesondere über einen geeigneten Pulsgeber, beispielsweise Hall-Sensor, erfasst werden. Es versteht sich, dass mit Hilfe des elektronischen Shutters zusätzlich auch die Belichtungszeit des Bildsensors gesteuert werden kann (also die Dauer der Belichtung). Grundsätzlich kann aber auch die rotierende Verschlussblende zur Steuerung der Belichtungszeit beitragen. Bei der Verschlussblende kann es sich um eine so genannte Sektorblende handeln, welche eine oder mehrere Segmentöffnungen aufweist.

Mit dem stereoskopischen Abbildungssystem erfolgt eine elektronische Erfassung der mittels des Objektivs abgebildeten Halbbilder, so dass die von dem Bildsensor generierten, in elektronischer Form vorliegenden Bilddaten oder Bildsignale beliebig aufgeteilt und/oder vervielfacht werden können. Somit ist es möglich, dass mehrere Personen gleichzeitig das von dem Abbildungssystem erfasste Objekt beobachten können, ohne dass es hierbei zu einer Abschwächung der Lichtintensität kommt. Auch eine simultane Aufzeichnung der stereoskopischen Halbbilder zu Dokumentationszwecken ist ohne Qualitätsverlust möglich, wobei sowohl Standbilder als auch Bewegtbilder aufgezeichnet werden können. Weiterhin können die vom Bildsensor erfassten stereoskopischen Halbbilder auf vielfältige, nachfolgend noch näher erläuterte Weise wiedergegeben werden.

Insbesondere ist es auch möglich, den elektronisch erfassten stereoskopischen Halbbildern weitere digitale Bilder, die von demselben Untersuchungsobjekt zum Beispiel mittels Computertomographie (CT), Magnetresonanztomographie (MRT) oder anderen bildgebenden Verfahren gewonnen wurden, pixelgenau zu überlagern.

Ferner können die erfassten Halbbilder vor ihrer Wiedergabe individuell und simultan oder quasi-simultan aufbereitet und/oder bearbeitet werden. So können zum Beispiel Nerven, Blutgefäße und/oder andere Bestandteile eines Organismus mithilfe einer spezifischen farblichen Hervorhebung dargestellt werden. Auch eine Helligkeits- und/oder Kontrastanpassung einzelner Bildbereiche ist möglich. Ferner kann eine elektronische Bildstabilisierung vorgesehen werden, um etwa Vibrationen oder Stöße bei der Bildaufnahme zu kompensieren und eine verwackelungsfreie Darstellung zu ermöglichen. Auch eine elektronische Kompensation von bekannten Farbfehlern des Objektivs kann vorgesehen werden.

Schließlich ist es bei dem stereoskopischen Abbildungssystem auch möglich, mithilfe von Triangulationsverfahren oder anderen geeigneten Verfahren zusätzlich zu den zweidimensionalen Bildinformationen quantitative Tiefeninformationen zu gewinnen, so dass letztlich dreidimensionale (3D-)Bilddaten in digitaler Form zur Verfügung stehen. Diese 3D-Bilddaten können z.B. zum elektronischen Vermessen auch von solchen Objektstrukturen verwendet werden, die sich nicht parallel zur Bildebene sondern geneigt hierzu erstrecken. Diese In-Bild-Vermessung kann auch intra-operativ, also etwa während eines laufenden Operationsvorgangs, durchgeführt werden und wahlweise durch eine strukturierte Beleuchtung oder durch Laserspots, die auf die Objektstrukturen projiziert werden, unterstützt werden.

Erfindungsgemäß ist das Abbildungssystem dazu angepasst, einen von wenigstens zwei vorbestimmten Spektralbereichen auszuwählen, welcher von dem Bildsensor zum Erfassen eines jeweiligen Paares von stereoskopischen Halbbildern berücksichtigt wird. Es können also zwei oder mehrere, sich überlappende oder nicht überlappende Spektralbereiche erfasst werden, beispielsweise zum einen ein sichtbarer Spektralbereich und zum anderen ein ultravioletter oder infraroter Spektralbereich. Die Auswahl des jeweiligen Spektralbereichs kann zum Beispiel durch entsprechende Filter erfolgen, die in der rotierenden Verschlussblende (oder an anderer Stelle im Abbildungsstrahlengang) vorgesehen sind. So kann beispielsweise ein Segmentausschnitt der Verschlussblende in einem Teilabschnitt einen Filter für den einen Spektralbereich und in einem anderen Teilabschnitt einen Filter für den anderen Spektralbereich aufweisen, so dass nacheinander zwei Halbbilder mit derselben Perspektive, aber unterschiedlichen Spektralbereichen aufgenommen werden. Weiterhin kann auch eine separate rotierende Filterblende eingesetzt werden, welche mit einer Drehzahl rotiert, die zu der Drehzahl der rotierenden Verschlussblende in einem geeigneten Verhältnis steht.

Zusätzlich kann eine Beleuchtungseinrichtung zur Erzeugung einer unterschiedlichen Beleuchtungscharakteristik angesteuert werden. So kann beispielsweise eine UV-Lampe zur Anregung von Fluoreszenz in dem zu beobachtenden Objekt nur dann angesteuert werden, wenn tatsächlich ein Halbbild im ultravioletten Spektralbereich aufgenommen werden soll. Somit ist eine noch bessere Trennung der Spektralbereiche bzw. Farbkanäle möglich.

Jedem Spektralbereich ist eine Bildgruppe zugeordnet, wobei das Abbildungssystem (insbesondere die genannte Synchronisations- und Steuereinrichtung) dazu angepasst ist, den Spektralbereich zeitlich alternierend auszuwählen und die Paare von stereoskopischen Halbbildern in Abhängigkeit von dem ausgewählten Spektralbereich der entsprechenden Bildgruppe zuzuordnen, so dass jeder Bildgruppe nur diejenigen vom Bildsensor erfassten Halbbilder zugeordnet sind, die gemäß demselben ausgewählten Spektralbereich erfasst worden sind. Die Zuordnung zu den Bildgruppen kann zum Beispiel durch entsprechende Flags (d.h. durch Kennungsdaten) erfolgen, die an die eigentlichen Bilddaten der jeweiligen Halbbilder angefügt sind.

Gemäß einer vorteilhaften Ausführungsform ist die Synchronisations- und Steuereinrichtung dazu angepasst, die Drehzahl der rotierenden Verschlussblende (d.h. die Rotationsfrequenz) an eine Bildfrequenz des Bildsensors anzupassen, die der eingestellten Ladungsintegrationszeit entspricht. Hierbei ist also die Bildfrequenz fest oder auch einstellbar entsprechend der Ladungsintegrationszeit vorgegeben, und die Drehzahl der Verschlussblende wird so angepasst (insbesondere durch eine geeignete Regelung), dass eine Synchronisation zwischen der Bildfrequenz und der rotierenden Verschlussblende gegeben ist.

Alternativ kann die Synchronisations- und Steuereinrichtung dazu angepasst sein, eine Bildfrequenz des Bildsensors, die der eingestellten Ladungsintegrationszeit entspricht, an eine vorgegebene Drehzahl der rotierenden Verschlussblende anzupassen. Auch hier kann die Drehzahl fest oder einstellbar sein. Die Synchronisation wird in dem Fall durch eine Anpassung der Ladungsintegrationszeit und damit der Bildfrequenz vorgenommen.

Gemäß einer weiteren vorteilhaften Ausführungsform ist die Doppelaperturblende derart angepasst, dass der Abstand der Aperturen und/oder die Größe der Aperturen verändert werden kann. Durch eine Variation des Abstands der Aperturen kann der Betrachtungswinkel auf das Objekt verändert werden, so dass je nach Einstellung ein unterschiedlich starker räumlicher Eindruck erzielt werden kann. Durch eine Anpassung der Größe der Aperturen können alternativ oder zusätzlich zu der Beeinflussung der Perspektive auch der Schärfentiefebereich und die Lichtstärke gesteuert werden. Alternativ kann auch die Doppelaperturblende auswechselbar sein. Hierbei ist es möglich, verschiedene Doppelaperturblenden mit unterschiedlichem Abstand der Aperturen und/oder unterschiedlicher Größe der Aperturen bereitzuhalten, um auf entsprechende Weise den Betrachtungswinkel und/oder den Schärfentiefebereich und die Lichtstärke zu variieren.

Ein besonderer Vorteil der Verwendung einer derartigen Doppelaperturblende besteht darin, dass die genannten optischen Parameter auf einfache Weise geändert werden können (beispielsweise durch Austauschen der Doppelaperturblende), wobei die Doppelaperturblende ein einfaches und kostengünstiges Bauteil sein kann. Die anderen Komponenten des Abbildungssystems können hingegen auf größtmögliche Lichtstärke und Lichtempfindlichkeit ausgelegt sein, so dass der Benutzer hinsichtlich der Anpassung des Abbildungssystems an die konkrete Anwendung einen großen Gestaltungsfreiraum genießt.

Der elektronische Bildsensor ist bevorzugt ein Großformatsensor, der die Halbbilder mit einer Bildfrequenz von wenigstens 50 Hz erfasst. Unter einem Großformatsensor (oder auch "Vollformatsensor") wird im Zusammenhang mit der Erfindung ein Bildsensor verstanden, dessen aktive Bildfläche derjenigen Bildfläche entspricht, die mittels einer analogen 35-mm-Filmkamera auf fotografischem Film aufgezeichnet werden kann. Je nach dem gewählten Aufzeichnungsformat kann ein solcher Großformatsensor eine aktive Bildfläche von mindestens 21,95 mm x 9,47 mm aufweisen. Ein geeigneter beispielhafter Bildsensor, welcher unter der Bezeichnung "ARRI ALEV III" kommerziell verwendet wird, weist eine aktive Matrixfläche von 2.280 x 1.620 Pixel mit einer Pixelgröße von 8,25 µm x 8,25 µm auf, woraus sich eine aktive Bildfläche von 23,76 mm x 13,365 mm ergibt.

Ein derartiger großformatiger Bildsensor ist in der elektronischen Mikroskopie nicht üblich, da generell versucht wird, unter Verwendung kleiner Aperturen und kleiner Bildsensoren einen großen Schärfentiefebereich vorzusehen. Im Zusammenhang mit der Erfindung wurde allerdings erkannt, dass ein großformatiger Bildsensor mit entsprechend vergrößerter Pixelgröße aufgrund der hierdurch erzielbaren hohen Lichtempfindlichkeit wichtige Anwendungen in der Mikroskopie erschließt, beispielsweise durch Verwendung hoher Bildfrequenzen, wie nachstehend noch erläutert wird. Angepasst an einen derartigen großformatigen Bildsensor können Objektive verwendet werden, die im Vergleich zu Abbildungssystemen, bei denen ein Bildsensor mit wesentlich kleinerer Bildfläche eingesetzt wird, bei gleichem Abbildungsmaßstab eine größere Brennweite aufweisen, was zu einer verbesserten Abbildungsqualität führt. Ein ausreichender Schärfentiefebereich kann im Bedarfsfall durch Wahl einer geeigneten Größe der Aperturen erhalten werden.

Desweiteren ermöglicht ein großformatiger Bildsensor auch die Erfassung eines größeren Sichtfeldes. Dies kommt insbesondere der Handhabbarkeit zu Gute und trägt damit zu einer Verkürzung der Operationszeit bei.

Vorzugsweise weist der Bildsensor eine Helligkeitsempfindlichkeit entsprechend wenigstens 14 Blendenstufen auf, um die erläuterten Vorteile einer hohen Lichtempfindlichkeit bei Anwendungen in der Mikroskopie zu erzielen.

Gemäß einer weiteren vorteilhaften Ausführungsform umfasst das Abbildungssystem ferner eine Beleuchtungseinrichtung, welche dazu angepasst ist, das Objekt alternierend aus zwei unterschiedlichen Beleuchtungswinkeln zu beleuchten, wobei jedem Beleuchtungswinkel einer der Teilstrahlengänge zugeordnet ist, und wobei die Synchronisations- und Steuereinrichtung ferner dazu angepasst ist, den jeweiligen Beleuchtungswinkel synchron zu dem Freigeben des zugeordneten Teilstrahlengangs zu aktivieren. Durch die alternierende Änderung des Beleuchtungswinkels kann der stereoskopische Effekt noch verstärkt werden.

Alternativ oder zusätzlich kann die Beleuchtungseinrichtung wenigstens eine erste Beleuchtungseinheit, welche eine nahe der optischen Achse angeordnete Lichtaustrittsstelle oder mehrere nahe der optischen Achse angeordnete Lichtaustrittsstellen aufweist, und wenigstens eine zweite Beleuchtungseinheit umfassen, deren Lichtaustrittsstelle oder Lichtaustrittsstellen fern der optischen Achse des Abbildungssystems angeordnet ist bzw. sind. Die zweite Beleuchtungseinheit kann z.B. zur Gesamtbeleuchtung des Operationsbereichs und/oder zur gezielten Erzeugung eines Schattenwurfs im Sichtfeld des Abbildungssystems dienen. Die genannte zweite Beleuchtungseinheit kann beispielsweise eine Ringleuchte mit mehreren selektiv aktivierbaren und deaktivierbaren Beleuchtungssegmenten umfassen, wobei durch Deaktivieren unterschiedlicher Beleuchtungssegmente ein Schattenwurf in verschiedenen Richtungen erzeugt werden kann, um bestimmte Bereiche im Sichtfeld genauer darzustellen.

Alternativ oder zusätzlich hierzu wiederum kann das Abbildungssystem eine Beleuchtungseinrichtung umfassen, die wenigstens ein Beleuchtungsstrahlbündel erzeugt, um das abzubildende Objekt zu beleuchten. Bei dieser Ausführungsform weist wenigstens eine Linse des Objektivs (insbesondere eine Linsengruppe des Objektivs) wenigstens eine Ausnehmung auf, durch die der Querschnitt der genannten Linsen sich von der üblichen Form einer geschlossenen Kreisfläche unterscheidet. Mit anderen Worten bildet die genannte Ausnehmung eine Abweichung des Querschnitts der jeweiligen Linse von einer Kreisflächenform. Das genannte wenigstens eine Beleuchtungsstrahlbündel verläuft bei dieser Ausführungsform entlang der optischen Achse des Objektivs innerhalb der genannten Ausnehmung der Linse.

Hierdurch ist es möglich, das jeweilige Beleuchtungsstrahlbündel in geringem Abstand entlang der optischen Achse des Objektivs in Richtung des abzubildenden Objekts zu führen, so dass das Objekt mit lediglich geringem Schattenwurf beleuchtet wird und eine kompakte Bauform für das Objektiv möglich ist. Ferner ermöglicht die genannte Ausnehmung eine ungehinderte Ausbreitung des jeweiligen Beleuchtungsstrahlbündels, d.h. ohne dass das Beleuchtungsstrahlbündel auf eine optische Grenzfläche der jeweiligen Linse trifft, wodurch ein unerwünschter Streulichteinfall in die optischen Elemente des Objektivs zuverlässig vermieden wird.

Die genannte wenigstens eine Ausnehmung der Linse kann eine beliebige Form aufweisen, solange nur das Beleuchtungsstrahlbündel innerhalb der Ausnehmung verläuft. Beispielsweise kann die Ausnehmung durch eine Abflachung, eine kreisförmige Bohrung oder eine kreissektorförmige Bohrung gebildet sein. Die genannte Ausnehmung kann an einem Randbereich der Linse oder innerhalb der Linse vorgesehen sein. Vorzugsweise erstrecken die genannte Ausnehmung und das jeweilige Beleuchtungsstrahlbündel sich entlang des Objektivs parallel zu der optischen Achse. Ferner ist es bevorzugt, wenn die genannte Ausnehmung entlang der optischen Achse betrachtet versetzt zu den beiden Aperturen der Doppelaperturblende ist. Hierdurch bewirkt die genannte Ausnehmung keine übermäßige Beeinträchtigung der optischen Abbildungseigenschaften der jeweiligen Linse bezüglich der beiden genannten, den Aperturen zugeordneten Teilstrahlengänge des Abbildungsstrahlengangs.

Bei dieser Ausführungsform kann die Beleuchtungseinrichtung wenigstens eine Zerstreuungslinse aufweisen, die das jeweilige Beleuchtungsstrahlbündel in Richtung des zu beleuchtenden und abzubildenden Objekts aufweitet. Die genannte Zerstreuungslinse ist bezogen auf die Ausbreitungsrichtung des Beleuchtungsstrahlbündels insbesondere hinter der die Ausnehmung aufweisenden Linse angeordnet, vorzugsweise hinter dem Objektiv oder hinter der letzten Linse des Objektiv (wiederum bezogen auf eine Betrachtung entlang der Ausbreitungsrichtung des Beleuchtungsstrahlbündels). Hierdurch wird erreicht, dass trotz des Aufweitens des Beleuchtungsstrahlbündels mittels der Zerstreuungslinse die Beleuchtung kein optisches Element des Objektivs beaufschlagt, was wie erläutert zu unerwünschten Streueffekten führen könnte.

Vorteilhafterweise kann das Abbildungssystem eine Beleuchtungseinrichtung zum Beleuchten des Objekts umfassen, bei der es sich insbesondere um eine der vorgenannten Beleuchtungseinrichtungen handeln kann, wobei die Synchronisations- und Steuereinrichtung dazu angepasst ist, die Beleuchtungseinrichtung alternierend (insbesondere paarweise alternierend) zur Erzeugung einer unterschiedlichen Beleuchtungscharakteristik anzusteuern. Der elektronische Bildsensor wird bei dieser Ausführungsform dazu angesteuert, die Halbbilder mit einer Bildfrequenz von wenigstens 50 Hz zu erfassen. Unter einer Beleuchtungscharakteristik kann beispielsweise ein unterschiedlicher Spektralbereich der Beleuchtung verstanden werden. So kann beispielsweise alternierend ein erstes Paar von Halbbildern mit einer Beleuchtung im sichtbaren Spektralbereich und wenigstens ein zweites Paar von Halbbildern mit einer Beleuchtung im infraroten und/oder ultravioletten Spektralbereich, etwa zur Anregung von fluoreszierenden Markern eines entsprechend präparierten Objekts, erzeugt werden. Dadurch können mehrere Farbkanäle im Zeitmultiplex-Verfahren aufgenommen werden, wobei die Bildfrequenz von wenigstens 50 Hz eine ausreichende Bildwiederholrate gewährleistet, die für eine möglichst flüssige Wiedergabe von Bewegtbildern erforderlich ist.

Vorzugsweise ist auch bei dieser Ausführungsform der Bildsensor als ein Großformatsensor ausgebildet, der aufgrund einer erhöhten Pixelgröße eine besonders hohe Lichtempfindlichkeit besitzen kann (vorzugsweise wenigstens 14 Blendenstufen). Eine erhöhte Lichtempfindlichkeit ergibt bei dieser Ausführungsform den Vorteil, dass die entsprechend der hohen Bildfrequenz verringerte Belichtungszeit des Bildsensors kompensiert wird, so dass die Halbbilder insgesamt mit einer Helligkeit erzeugt werden, die für eine genaue Beobachtung des Objekts durch den Benutzer (z.B. Operateur) ausreichend ist.

Ferner ermöglicht die Verwendung eines Großformatsensors mit erhöhter Lichtempfindlichkeit eine Reduktion der zur Beleuchtung des Objekts erforderlichen Lichtmenge, woraus sich zwei wesentliche Vorteile ergeben. Zum einen ist der Blickwechsel des Operateurs vom mikroskopischen Bild zum direkten Betrachten des Operationsfeldes mit weniger Adaptionstätigkeit des Auges verbunden, was wiederum zu einer Verkürzung der Operationszeit führen kann. Zum anderen vermindert die reduzierte Lichtmenge die Gefahr, dass die entstehende Wärme der auftreffenden Lichtenergie, insbesondere bei der Verwendung von Xenon-Lichtquellen oder dergleichen, das Gewebe des Patienten stark austrocknet. Gerade bei zeitintensiven Eingriffen ohne kontinuierliche Spülung, z.B. bei der Gefäß-oder Neurochirurgie, könnte dies ansonsten unter Umständen zu einer irreversiblen Nekrose beitragen.

Gemäß einer weiteren vorteilhaften Ausgestaltung weist das Abbildungssystem wenigstens ein Wiedergabesystem zum Wiedergeben der erzeugten Halbbilder auf, das mit einem Ausgang des Aufnahmesystems elektronisch, nicht jedoch nach Art eines herkömmlichen Stereomikroskops optisch verbunden ist. Dadurch können das Aufnahmesystem und das Abbildungssystem mechanisch voneinander entkoppelt sein. Gegenüber einem herkömmlichen, rein optischen Abbildungssystem wird dadurch vermieden, dass Stöße, die der Benutzer beispielsweise über das Okularpaar versehentlich auf das Abbildungssystem ausübt, zu einer verwackelten Bilddarstellung führen oder sich gar auf das Objekt übertragen. Zudem kann der Benutzer, beispielsweise der Operateur bei einer Anwendung der Erfindung bei einem Operationsmikroskop, eine wesentlich flexiblere Arbeitsposition einnehmen, als es im Vergleich zu einem rein optischen System möglich ist. Die dauerhafte Gewährleistung einer ergonomisch günstigen Sitzposition verringert Ermüdungserscheinungen und erhöht somit die Handhabungsgenauigkeit der chirurgischen Instrumente durch den Operateur.

Gemäß einer vorteilhaften Ausführungsform kann das Wiedergabesystem einen einzigen Monitor zum Darstellen der erzeugten Halbbilder und ein Paar von Okularen aufweisen, wobei das Wiedergabesystem ferner eine Strahlteilungseinrichtung umfasst, welche die gemäß dem einen Strahlengang erzeugten Halbbilder zu dem einen Okular und die gemäß dem anderen Teilstrahlengang erzeugten Halbbilder zu dem anderen Okular lenkt. Eine derartige Strahlteilungseinrichtung kann beispielsweise eine rotierende Blende in Kombination mit einer Umlenkeinrichtung oder einen rotierenden Segmentspiegel umfassen, welche das von dem Monitor dargestellte Bild wechselweise zu einem der beiden Okulare lenken. Weiterhin kann die Strahlteilungseinrichtung auch einen Strahlteiler umfassen, welches den Wiedergabestrahlengang in zwei Wiedergabe-Teilstrahlengänge aufteilt, wobei jeder Wiedergabe-Teilstrahlengang durch einen mechanischen oder elektronischen Shutter, beispielsweise durch einen LCD-Shutter, wechselweise freigegeben bzw. unterbrochen wird.

Alternativ kann das Wiedergabesystem zwei Monitore zum Darstellen der erzeugten Halbbilder aufweisen, wobei jedem Monitor ein Okular zugeordnet ist und wobei der eine Monitor zur Wiedergabe der von dem einen Teilstrahlengang erzeugten Halbbilder und der andere Monitor zur Wiedergabe der von dem einen Teilstrahlengang erzeugten Halbbilder vorgesehen ist.

Ferner kann das Wiedergabesystem auch einen einzigen Monitor zur direkten, also okularfreien Betrachtung durch den Benutzer aufweisen, wobei zeitlich alternierend Halbbilder der Links- bzw. Rechtsperspektive dargestellt werden und eine vom Benutzer zu tragende Shutterbrille synchron zu dieser Darstellung alternierend das der jeweiligen Perspektive zugeordnete Auge des Benutzers für eine Betrachtung des Monitors freigibt. Alternativ kann der Monitor auch als 3D-Monitor auf Polarisationsbasis oder als 3D-Monitor auf autostereoskopischer Basis ausgeführt sein.

Ein Vorteil des erfindungsgemäßen Abbildungssystems besteht insbesondere darin, dass auch mehrere der genannten Wiedergabesysteme simultan zum Einsatz gelangen können, z.B. eine optoelektronische binokulare Beobachtung durch den Operateur und gleichzeitig eine Darstellung an einem LCD-Bildschirm für einen Assistenten oder eine OP-Schwester.

Wie bereits erwähnt kann das stereoskopische Abbildungssystem für ein elektronisches Vermessen von tiefenabhängigen Abständen an dem abgebildeten Objekt verwendet werden, insbesondere wenn das Abbildungssystem ein Wiedergabesystem nach einer der vorstehend erläuterten Ausführungsformen umfasst. Hierzu ist es bevorzugt, wenn das Abbildungssystem ferner eine Eingabeeinrichtung umfasst, die es einem Benutzer (z.B. dem Operateur) ermöglicht, einen jeweiligen ersten Bildmarkierungspunkt und einen jeweiligen zweiten Bildmarkierungspunkt innerhalb der Halbbilder festzulegen, die an dem Wiedergabesystem paarweise wiedergegeben werden. Der jeweilige erste Bildmarkierungspunkt entspricht hierbei an dem abgebildeten Objekt einem ersten Objektmarkierungspunkt, und der jeweilige zweite Bildmarkierungspunkt entspricht an dem abgebildeten Objekt einem zweiten Objektmarkierungspunkt, wobei diese beiden Objektmarkierungspunkte entlang der optischen Achse des Abbildungssystems in unterschiedlicher Tiefe (z-Koordinate) angeordnet sein können. Ferner umfasst das Abbildungssystem bei dieser Ausführungsform eine Auswerte- und Steuereinrichtung (als Teil der bereits genannten Synchronisations- und Steuereinrichtung oder separat hiervon), die dazu angepasst ist, einen tiefenabhängigen Abstand zwischen dem genannten ersten Objektmarkierungspunkt und dem genannten zweiten Objektmarkierungspunkt zu berechnen. Mit anderen Worten berechnet die Auswerte- und Steuereinrichtung die Länge der Verbindungslinie zwischen den zwei ausgewählten Objektmarkierungspunkten, wobei diese Verbindungslinie im Raum beliebig ausgerichtet sein kann. Zusätzlich kann die Auswerte- und Steuereinrichtung das Wiedergabesystem (direkt oder indirekt) dazu anzusteuern, die vom Benutzer festgelegten und den beiden Objektmarkierungspunkten entsprechenden Bildmarkierungspunkte darzustellen, beispielsweise durch Überlagerung von graphischen Symbolen auf den paarweise wiedergegebenen Halbbildern.

Die Berechnung des tiefenabhängigen Abstands kann bei dieser Ausführungsform beispielsweise nach einem Verfahren erfolgen, das die inhärente Disparität der beiden Halbbilder aufgrund der unterschiedlichen stereoskopischen Perspektive ausnutzt. Die Bildinhalte der beiden Halbbilder eines jeweiligen Paars von Halbbildern sind nämlich sehr ähnlich zueinander, so dass verschiedene Bildbereiche der beiden Halbbilder leicht einander zugeordnet werden können. Die jeweilige Position der einander entsprechenden Bildbereiche unterscheidet sich jedoch zwischen den beiden Halbbildern eines Paars nicht nur in Abhängigkeit von der jeweiligen Perspektive, sondern auch in Abhängigkeit von der Tiefe (z-Koordinate) des abgebildeten Objekts, d.h. die einander entsprechenden Bildbereiche werden in Abhängigkeit von der jeweiligen Perspektive und der Tiefe durch unterschiedliche Bildpunktpositionen innerhalb der beiden Halbbilder dargestellt. Die Abhängigkeit von der jeweiligen Perspektive ist aus der Geometrie der Anordnung (z.B. Abstand der beiden Aperturen der Doppelaperturblende, eingestellter Zoomfaktor) bekannt und somit berechenbar. Aus den Abweichungen von den somit berechenbaren Unterschieden zwischen den beiden Halbbildern eines jeweiligen Paares kann somit die Tiefeninformation gewonnen werden. Dieses Verfahren hat den Vorteil, dass ausgehend von der ohnehin vorhandenen Bildinformation (Paare von Halbbildern entsprechend einer Rechts-Perspektive und einer Links-Perspektive) grundsätzlich keine zusätzlichen Komponenten zwingend erforderlich sind, insbesondere keine zusätzliche Lichtsendeeinrichtung oder Lichtempfangseinrichtung mit zugeordneter Optik.

Alternativ zu einem derartigen Disparitätsverfahren kann beispielsweise im Bereich des dem ersten oder zweiten Objektmarkierungspunkt entsprechenden Bildmarkierungspunkts die Modulationsübertragungsfunktion (MTF, Modulation Transfer Function) innerhalb des jeweiligen Halbbilds gemessen und ausgewertet werden, wobei zwischen diesen Messungen die Bildweite des Objektivs variiert wird (z.B. durch Versetzen eine Fokuslinse). Diejenige Bildweite, die die höchste MTF ergibt, entspricht dem gesuchten Abstand des jeweiligen Objektmarkierungspunkts. Dieser Abstand resultiert für eine gegebene Bildweite unmittelbar aus den bekannten Eigenschaften des Objektivs (entsprechende Gegenstandsweite).

Als weitere Alternative für die Berechnung des Abstands eines ausgewählten Objektmarkierungspunkts von dem Abbildungssystem kann ein Triangulationsverfahren zum Einsatz gelangen, das jedoch typischerweise den Einsatz einer zusätzlichen Lichtsendeeinrichtung und einer zusätzlichen Lichtempfangseinrichtung mit zugeordneter Optik erfordert.

Gemäß einer vorteilhaften Ausführungsform kann die genannte Eingabeeinrichtung (zum Festlegen des ersten Bildmarkierungspunkts und des zweiten Bildmarkierungspunkts) eine berührungsempfindliche Oberfläche des Wiedergabesystems umfassen. Beispielsweise kann die Oberfläche des Wiedergabesystems dazu angepasst sein, durch Messung einer lokalen Änderung einer Kapazität oder eines elektrischen Widerstands eine Ortsinformation zu erzeugen (so genannter kapazitiver oder resistiver Touch Screen). Eine derartige Ausführungsform ermöglicht eine besonders schnelle und intuitiv eingängliche Bedienung. Alternativ hierzu kann die genannte Eingabeeinrichtung jedoch beispielsweise auch einen verschwenkbaren Steuerknüppel und eine zugeordnete Auswahltaste umfassen.

Insbesondere bei einem rein passiven System (beispielsweise bei dem genannten Disparitätsverfahren oder MTF-Verfahren ohne Verwendung einer zusätzlichen Lichtsendeeinrichtung) kann das Abbildungssystem gemäß einer vorteilhaften Ausführungsform ferner einen Bildgenerator umfassen (z.B. als Teil der Auswerte-und Steuereinrichtung oder separat hiervon), der den an dem Wiedergabesystem wiedergegebenen Halbbildern eine Wiedergabe eines jeweiligen Markierungspunktsymbols überlagert (z.B. Kreuz-Symbol). Die genannte Eingabeeinrichtung ist bei dieser Ausführungsform dazu angepasst, einen Markierungspunktauswahlbefehl des Benutzers (z.B. Operateur) zu erfassen und ein entsprechendes Markierungspunktauswahlsignal zu erzeugen. In Abhängigkeit von dem von der Eingabeeinrichtung erzeugten Markierungspunktauswahlsignal (z.B. wenn der Benutzer eine berührungsempfindliche Oberfläche antippt oder eine Taste drückt) steuert die Auswerte- und Steuereinrichtung den Bildgenerator dazu an, das jeweilige Markierungspunktsymbol an einer dem Markierungspunktauswahlsignal entsprechenden Position an dem Wiedergabesystem wiederzugeben. Beispielsweise kann das Markierungspunktauswahlsignal selbst eine Ortsinformation enthalten, oder die Ortinformation ergibt sich indirekt aus einer vorherigen Befehlseingabe. Ferner ordnet die Auswerte- und Steuereinrichtung die momentane Position des Markierungspunktsymbols einer jeweiligen Bildpunktposition innerhalb der an dem Wiedergabesystem wiedergegebenen Halbbilder zu, wobei diese zugeordnete Bildpunktposition den jeweiligen ersten Bildmarkierungspunkt oder den jeweiligen zweiten Bildmarkierungspunkt für die Abstandsberechnung bildet. Mit anderen Worten wird durch diese Zuordnung der jeweilige erste Bildmarkierungspunkt oder der jeweilige zweite Bildmarkierungspunkt für die Abstandsberechnung festgelegt. Da jedes der von dem Aufnahmesystem erzeugten Bilder aus zwei Halbbildern besteht, werden hierbei für den ersten Bildmarkierungspunkt oder den zweiten Bildmarkierungspunkt jeweils eine Bildpunktposition innerhalb des einen Halbbildes und eine Bildpunktposition innerhalb des anderen Halbbildes des Paares von Halbbildern festgelegt. Somit wird dem Benutzer bei dieser Ausführungsform eine in die Wiedergabe der Halbbilder integrierte graphische Bedieneroberfläche zu Verfügung gestellt, um auf einfache Weise die beiden Bildmarkierungspunkte für die Abstandsmessung definieren zu können.

Optional kann bei dieser Ausführungsform die Eingabeeinrichtung auch einen Positionseingabebefehl des Benutzers erfassen und ein entsprechendes Positionseingabesignal erzeugen. In diesem Fall steuert die Auswerte- und Steuereinrichtung in Abhängigkeit von dem von der Eingabeeinrichtung erzeugten Positionseingabesignal (z.B. wenn der Benutzer über eine berührungsempfindliche Oberfläche streicht, eine berührungsempfindliche Oberfläche antippt oder einen Steuerknüppel verschwenkt) den Bildgenerator dazu an, an einer bestimmten Position oder an einer geänderten Position innerhalb der wiedergegebenen Halbbilder, die dem von der Eingabeeinrichtung erzeugten Positionseingabesignal entspricht, ein jeweiliges Positionsanzeigesymbol an dem Wiedergabesystem wiederzugeben. Auf diese Weise kann während eines Abstandsvermessungsbetriebsmodus des Abbildungssystems beispielsweise ständig ein Positionsanzeigesymbol (z.B. Kreis-Symbol) wiedergegeben werden, dessen Position innerhalb der an dem Wiedergabesystem wiedergegebenen Halbbilder geändert werden kann, bevor durch die Eingabe des genannten Markierungspunktauswahlbefehls die Position des jeweiligen Bildmarkierungspunkt endgültig festgelegt wird und das genannte Markierungspunktsymbol (z.B. Kreuz-Symbol) dargestellt wird.

Um mit einem lediglich geringen Zusatzaufwand die Genauigkeit der Abstandsmessung (beispielsweise des genannten Disparitätsverfahren oder MTF-Verfahrens) zu verbessern und die Bedienungsfreundlichkeit zu erhöhen, kann gemäß einer vorteilhaften Ausführungsform das Abbildungssystem ferner eine Markierungsstrahl-Sendeeinrichtung umfassen, die einen Markierungslichtstrahl aussendet (vorzugsweise ein kollimiertes Bündel sichtbaren Lichts). Hierdurch wird an dem abgebildeten Objekt ein Lichtfleck erzeugt, der somit auch innerhalb der von dem Bildsensor erfassten und an dem Wiedergabesystem wiedergegebenen Halbbilder für den Benutzer gut sichtbar ist. Die genannte Eingabeeinrichtung ist bei dieser Ausführungsform dazu angepasst, einen Richtungsänderungsbefehl des Benutzers zu erfassen und ein entsprechendes Richtungssteuersignal zu erzeugen sowie einen Markierungspunktauswahlbefehl des Benutzers zu erfassen und ein entsprechendes Markierungspunktauswahlsignal zu erzeugen. In Abhängigkeit von dem von der Eingabeeinrichtung erzeugten Richtungssteuersignal (z.B. wenn der Benutzer einen Steuerknüppel verschwenkt) ändert die Markierungsstrahl-Sendeeinrichtung die Ausrichtung des Markierungslichtstrahls (z.B. durch entsprechende Ansteuerung mittels der Auswerte- und Steuereinrichtung). Hierdurch wird die Position des Lichtflecks an dem Objekt geändert. In Abhängigkeit von dem von der Eingabeeinrichtung erzeugten Markierungspunktauswahlsignal (z.B. wenn der Benutzer eine Taste gedrückt hat) ordnet die Auswerte- und Steuereinrichtung die momentane Position des Lichtflecks an dem Objekt einer jeweiligen Bildpunktposition innerhalb der an dem Wiedergabesystem wiedergegebenen Halbbilder zu. Hierdurch wird der jeweilige erste Bildmarkierungspunkt oder der jeweilige zweite Bildmarkierungspunkt für die Abstandsberechnung festgelegt. Somit kann der Benutzer bei dieser Ausführungsform einen besonders deutlich sichtbaren Lichtfleck an dem Objekt erzeugen, um die beiden Bildmarkierungspunkte für die Abstandsmessung auf einfache und besonders zuverlässige Weise definieren zu können. Durch das Projizieren eines Lichtflecks auf diejenige Stelle, die als Objektmarkierungspunkt ausgewählt werden soll, wird ferner die Zuverlässigkeit der Abstandsmessung erhöht, da ein besserer Kontrast zu der Bildumgebung (Helligkeit und/oder Farbe) gewährleistet ist.

Alternativ oder zusätzlich zu dem genannten Wiedergabesystem kann das Abbildungssystem ferner eine Aufzeichnungseinrichtung zum Aufzeichnen der von dem Bildsensor erfassten Halbbilder aufweisen, die mit einem Ausgang des Bildsensors des Aufnahmesystems elektronisch verbunden ist. Somit ist unabhängig von der Wiedergabe der Halbbilder eine Aufzeichnung der Halbbilder zu Dokumentationszwecken in Form von digitalen Stand- und/oder Bewegtbildern auf einem geeigneten Speichermedium möglich.

Ferner kann das Abbildungssystem wenigstens eine Datenübertragungsschnittstelle zum Fernübertragen der erzeugten Halbbilder über ein Datennetz aufweisen, die einen elektronischen Ausgang des Bildsensors des Aufnahmesystems bildet. Das Fernübertragen kann beispielsweise über ein Intranet oder über das Internet erfolgen, so dass etwa ein Operationsvorgang nicht nur von dem durchführenden Operateur, sondern zusätzlich auch von einem eventuell weit entfernten Spezialisten, z.B. einem Pathologen während einer Tumorentfernung, simultan beobachtet werden kann.

Eine besonders vorteilhafte synergetische Kombination der vorgenannten Ausführungsformen liegt vor, wenn das Aufnahmesystem wenigstens einen ersten Datenausgang aufweist, der dazu angepasst ist, die mittels des Bildsensors erfassten Halbbilder an ein Wiedergabesystem der erläuterten Art zum Wiedergeben der Halbbilder auszugeben. Das Aufnahmesystem kann zusätzlich wenigstens einen zweiten Datenausgang aufweisen, der dazu angepasst ist, die erfassten Halbbilder an eine Aufzeichnungseinrichtung der erläuterten Art zum Aufzeichnen der Halbbilder auszugeben und/oder die erzeugten Halbbilder an ein Datennetz zum Fernübertragen der Halbbilder auszugeben. Ferner umfasst das Abbildungssystem eine Beleuchtungseinrichtung zum Beleuchten des Objekts gemäß einer variablen Beleuchtungscharakteristik. Die Synchronisations- und Steuereinrichtung ist bei dieser Ausführungsform dazu angepasst, den elektronischen Bildsensor zum Erfassen der Halbbilder mit einer Bildfrequenz von wenigstens 50 Hz anzusteuern und die Beleuchtungseinrichtung synchron hierzu alternierend (z.B. paarweise alternierend) zur Erzeugung wenigstens einer ersten Beleuchtungscharakteristik und einer hiervon verschiedenen zweiten Beleuchtungscharakteristik anzusteuern. Die Synchronisations- und Steuereinrichtung steuert den Bilddatenfluss so, dass an dem ersten Datenausgang ein erster Strom von Paaren von Halbbildern und an dem zweiten Datenausgang ein zweiter Strom von Paaren von Halbbildern ausgegeben werden, wobei die beiden Ströme von Halbbildern sich hinsichtlich der für die Erzeugung der Halbbilder verwendeten jeweiligen Beleuchtungscharakteristik teilweise oder vollständig voneinander unterscheiden.

Beispielsweise kann der erste Strom von Paaren von Halbbildern nur solche Halbbilder umfassen, die gemäß der ersten Beleuchtungscharakteristik erzeugt worden sind, während der zweite Strom von Paaren von Halbbildern solche Halbbilder umfasst, die gemäß der zweiten Beleuchtungscharakteristik oder gemäß der ersten und der zweiten Beleuchtungscharakteristik erzeugt worden sind. In entsprechender Weise können auch drei oder noch mehr Ströme von Paaren von Halbbildern erzeugt werden, die mehreren verschiedenen, alternierenden Beleuchtungscharakteristiken entsprechen.

Insbesondere ist es auch möglich, zwei, drei oder noch mehr Ströme von Paaren von Halbbildern zu erzeugen, die mehreren verschiedenen getrennten oder überlappenden sichtbaren Spektralbereichen entsprechen. Hierfür kann beispielsweise die Beleuchtungscharakteristik entsprechend variiert werden, während ein für das gesamte Beleuchtungsspektrum empfindlicher Bildsensor verwendet wird. Auf diese Weise können mehrere sichtbare Farbkanäle gebildet werden, um entsprechende spektral unterschiedliche Paare von Halbbildern zu erfassen (z.B. Rot, Grün und Blau), die erst nachträglich in einer jeweils gewünschten Auswahl oder Gewichtung miteinander kombiniert werden.

Somit ist es bei dieser Ausführungsform möglich, an wenigstens zwei verschiedenen Datenausgängen simultan zwei oder mehr Datenströme von stereoskopischen Halbbildern auszugeben, die einer unterschiedlichen Beleuchtungscharakteristik entsprechen. Die Erzeugung der verschiedenen Ströme von Halbbildern (entsprechend mehreren Farbkanälen) erfolgt also in einem Zeitmultiplex-Verfahren. Beispielsweise kann ein erster Strom von Paaren von Halbbildern basierend auf einer Beleuchtungscharakteristik eines sichtbaren Spektralbereichs erzeugt werden, und simultan (z.B. jeweils um ein Paar von Halbbildern zeitlich versetzt) kann ein zweiter Strom von Paaren von Halbbildern basierend auf einer Beleuchtungscharakteristik eines nicht-sichtbaren Spektralbereichs erzeugt werden (z.B. Fluoreszenzanregung im Ultravioletten oder Blutflussbeobachtung unter Infrarotbeleuchtung). Eine mit dem zweiten Datenausgang gekoppelte Aufzeichnungseinrichtung kann beide Datenströme aufzeichnen. Der Benutzer beobachtet über ein mit dem ersten Datenausgang verbundenes Wiedergabesystem jedoch lediglich die basierend auf der Beleuchtungscharakteristik des sichtbaren Spektralbereichs erzeugten Halbbilder (erster Datenstrom).

Durch Verwendung einer hohen Bildfrequenz von 50 Hz oder mehr können die Sequenzen von stereoskopischen Halbbildern beider Ströme flüssig beobachtet werden. Wenn zusätzlich ein großformatiger Sensor, insbesondere ein Großformatsensor, mit einer entsprechend hohen Lichtempfindlichkeit verwendet wird, macht sich die erforderliche hohe Bildfrequenz (kurze Belichtungszeiten) hinsichtlich der Helligkeit nicht negativ bemerkbar. Da die Beobachtungskanäle lediglich elektronisch, nicht jedoch optisch getrennt werden, ist eine parallele Beobachtung, Aufzeichnung und/oder Fernübertragung verschiedener spektraler Kanäle ohne Einbuße von Bildinformation oder Helligkeit möglich.

Im Zusammenhang mit der Erfindung ist es von Vorteil, wenn das Abbildungssystem ferner eine Auswahleinrichtung zur Auswahl wenigstens einer der genannten Bildgruppen aufweist, welche dazu angepasst ist, das Abbildungssystem so zu steuern, dass nur die der ausgewählten Bildgruppe zugeordneten Halbbilder wiedergegeben werden. Die Auswahl kann beispielsweise mit Hilfe der genannten Flags erfolgen. Dadurch kann eine aufwendige Synchronisation zwischen dem Aufnahmesystem und dem Wiedergabesystem entfallen. Auch bei einer nachträglichen Betrachtung, beispielsweise bei einer Wiedergabe der aufgezeichneten Bilddaten, ist eine eindeutige Auswahl des zu betrachtenden Spektralbereichs möglich. Die Auswahl der Bildgruppen bei simultaner Betrachtung (Live-Bild) oder bei einer nachträglichen Betrachtung kann derart erfolgen, dass jeweils nur ein bestimmter Spektralbereich wiedergegeben wird oder aber auch derart, dass mehrere Spektralbereiche ausgewählt werden und eine elektronische Überlagerung der entsprechenden Bilddaten erfolgt. Die Überlagerung der Bildgruppen mehrerer Spektralbereiche in demselben Live-Bild ist bei herkömmlichen Mikroskopen nicht möglich.

Gemäß einer weiteren vorteilhaften Ausführungsform weist das Abbildungssystem einen Positionssensor auf, welcher der rotierenden Verschlussblende zugeordnet ist. Der Positionssensor ist dazu angepasst, die Winkelposition der rotierenden Verschlussblende zu ermitteln und der ermittelten Winkelposition entsprechende Positionssignale an die Synchronisations- und Steuereinrichtung zu übermitteln. Die Positionssignale enthalten (direkt oder indirekt) Informationen darüber, welcher der Teilstrahlengänge momentan freigegeben oder unterbrochen ist. Die Synchronisations- und Steuereinrichtung ist bei dieser Ausführungsform ferner dazu angepasst, auf der Grundlage der übermittelten Positionssignale jeweilige Kanalkennungen derart zu erzeugen und mit den vom Bildsensor erfassten Halbbildern zu verknüpfen, dass jedes Halbbild anhand der hiermit verknüpften Kanalkennung dem für das Erfassen dieses Halbbildes freigegebenen Teilstrahlengang zugeordnet ist. Somit kann jedes Halbbild auch nachträglich eindeutig dem entsprechenden Betrachtungswinkel, d.h. der entsprechenden linken oder rechten Stereoperspektive des Betrachters und damit bei der Wiedergabe auch dem entsprechenden Monitor bzw. Okular zugeordnet werden.

Weitere vorteilhafte Ausführungsformen der Erfindung sind in den Unteransprüchen, der Beschreibung und den Zeichnungen genannt.

Die Erfindung wird nachfolgend anhand von Ausführungsbeispielen mit Bezug auf die Zeichnung erläutert. Gleiche oder gleichartige Elemente sind darin mit denselben Bezugszeichen gekennzeichnet.
- Fig. 1: zeigt eine schematische Draufsicht eines stereoskopischen Abbildungssystems gemäß einem Ausführungsbeispiel.
- Fig. 2: zeigt eine Doppelaperturblende des stereoskopischen Abbildungssystems gemäß Fig. 1 in einer Frontansicht.
- Fig. 3: zeigt eine rotierende Verschlussblende des stereoskopischen Abbildungssystems gemäß Fig. 1 in einer Frontansicht.
- Fig. 4: zeigt eine schematische Seitenansicht eines stereoskopischen Abbildungssystems gemäß einem weiteren Ausführungsbeispiel.
- Fig. 5: zeigt eine Linsengruppe des stereoskopischen Abbildungssystems gemäß Fig. 4 in einer Frontansicht.
- Fig. 6: zeigt eine schematische Seitenansicht eines Teils eines Objektivs eines stereoskopischen Abbildungssystems und ein zugehöriges Blockschaltbild.
- Fig. 7: zeigt ein Blockschaltbild von Teilen eines stereoskopischen Abbildungssystems gemäß einem weiteren Ausführungsbeispiel.

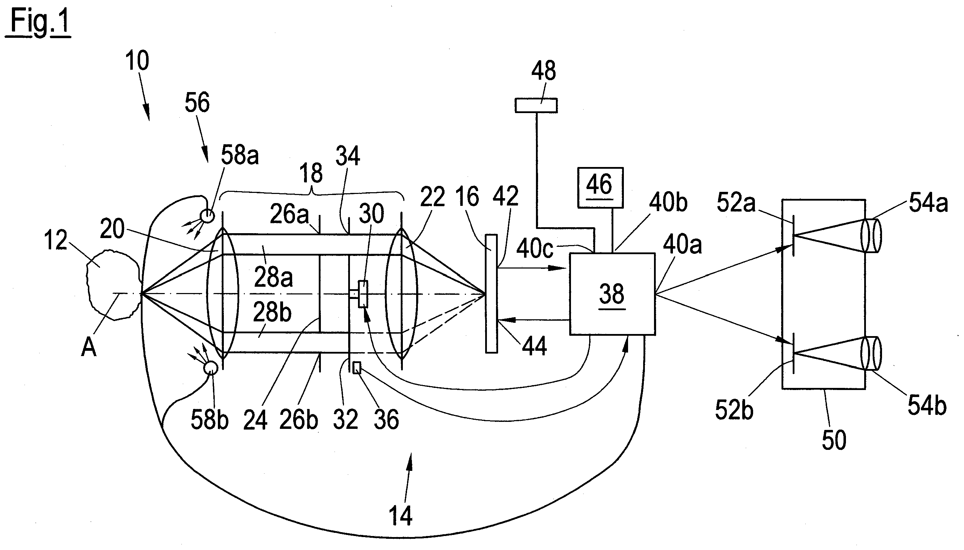

Ein stereoskopisches Abbildungssystem 10 zur Erzeugung von Paaren von stereoskopischen Halbbildern eines Objekts 12 umfasst ein Aufnahmesystem 14, welches einen elektronischen Bildsensor 16, beispielsweise einen CMOS-Sensor, und ein Objektiv 18 zum Erzeugen eines Abbildes des Objekts 12 auf einem Bildsensor 16 aufweist. Das Objektiv 18 umfasst in einer in Fig. 1 dargestellten vereinfachten Ausführungsform eine Frontlinse oder erste Linse 20 und eine Rücklinse oder zweite Linse 22. Die Linsen 20, 22 sind längs einer zentralen optischen Achse A angeordnet und bilden einen einzigen Abbildungsstrahlengang, dessen Strahlen im dargestellten Ausführungsbeispiel zwischen der ersten Linse 20 und der zweiten Linse 22 parallel verlaufen. Es versteht sich, dass auch komplexere mehrlinsige Objektive mit fester oder variabler Brennweite verwendet werden können. Ferner können auch spezielle Ausführungen von Objektiven verwendet werden, die aufgrund der verwendeten Glassorten, ihrer Apertur und/oder ihres Strahlengangs Anti-Aliasing-Eigenschaften aufweisen, so dass auf einen optischen Tiefpassfilter am Bildsensor verzichtet werden kann.

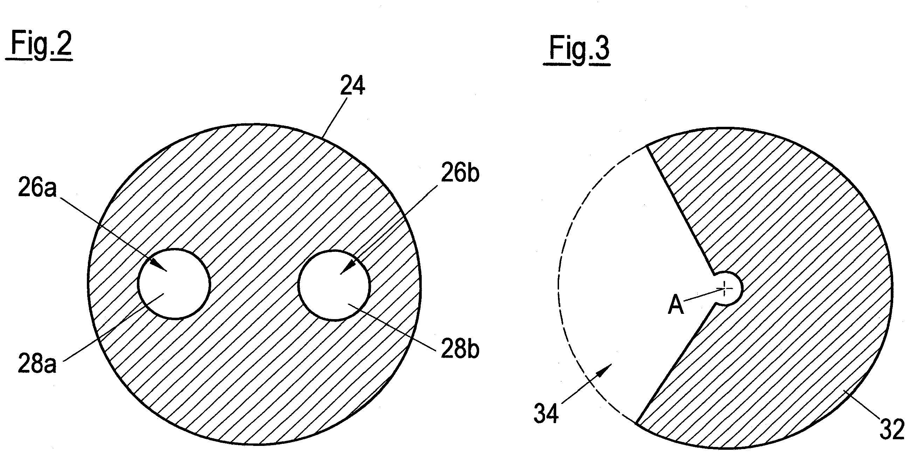

Zwischen den beiden Linsen 20, 22 ist eine feststehende Doppelaperturblende 24 angeordnet, welche zwei voneinander beabstandete und zu der optischen Achse A versetzte Aperturen 26a, 26b aufweist, die den Abbildungsstrahlengang in jeweilige Teilstrahlengänge 28a, 28b unterteilen. Die Doppelaperturblende 24 ist in einer Frontansicht auch in Fig. 2 gezeigt. Die Teilstrahlengänge 28a, 28b unterscheiden sich hinsichtlich ihres Betrachtungswinkels des Objekts 12, wobei der Teilstrahlengang 28a beispielsweise einer Rechts-Perspektive und der Teilstrahlengang 28b einer Links-Perspektive entsprechen kann.

Zwischen der Doppelaperturblende 24 und der zweiten Linse 22 ist eine um die optische Achse A rotierende, von einer Antriebsvorrichtung 30 angetriebene Verschlussblende 32 angeordnet, welche eine Segmentöffnung 34 aufweist, die derart ausgestaltet ist, dass die rotierende Verschlussblende 32 in Abhängigkeit von ihrer Winkelposition eine der beiden Aperturen 26a, 26b freigibt und die andere der beiden Aperturen 26a, 26b verschließt. Die Verschlussblende 32 ist in einer Frontansicht auch in Fig. 3 gezeigt. In der in Fig. 1 dargestellten Winkelposition ist die Apertur 26a freigegeben und die Apertur 26b verschlossen, so dass lediglich der Teilstrahlengang 28a von dem Bildsensor 16 erfasst werden kann, während der Teilstrahlengang 28b durch die Verschlussblende 32 unterbrochen ist und daher im weiteren Verlauf bis zum Bildsensor 16 nur gestrichelt dargestellt ist.

Der Verschlussblende 32 ist ein Positionssensor 36 zugeordnet, welcher die Winkelposition der Verschlussblende 32 ermittelt und entsprechende Positionssignale erzeugt.

Das Aufnahmesystem 14 weist ferner eine Synchronisations- und Steuereinrichtung 38 auf, welche mit dem Bildsensor 16, der Antriebsvorrichtung 30 und dem Positionssensor 36 verbunden ist. Obwohl die Synchronisations- und Steuereinrichtung 38 im vorliegenden Ausführungsbeispiel als eine einzige Einheit dargestellt ist, erfüllt sie mehrere Funktionen, die in alternativen Ausführungsbeispielen auch von mehreren separaten Untereinheiten erfüllt werden können.

Die von dem Bildsensor 16 erzeugten Bilddaten werden an einem Sensorausgang 42 bereitgestellt und an die Synchronisations- und Steuereinrichtung 38 übermittelt. Die Synchronisations- und Steuereinrichtung 38 erzeugt Steuersignale für einen elektronischen Shutter des Bildsensors 16 zum Einstellen der Bildfrequenz und wahlweise auch der Belichtungszeit des Bildsensors 16, welche an einen entsprechenden Sensoreingang 44 übermittelt werden. Die Synchronisations- und Steuereinrichtung 38 empfängt ferner die von dem Positionssensor 36 erzeugten Positionssignale und übermittelt Steuersignale zum Einstellen der Drehzahl der Verschlussblende 32 an die Antriebsvorrichtung 30.

Mit Hilfe der Synchronisations- und Steuereinrichtung 38 werden die durch den elektronischen Shutter des Bildsensors 16 eingestellte Bildfrequenz und die Drehzahl der rotierenden Verschlussblende 32 derart miteinander synchronisiert, dass der Bildsensor 16 während einer Zeitperiode, in welcher eine der Aperturen 26a, 26b freigegeben ist, ein jeweiliges stereoskopisches Halbbild auf der Grundlage des freigegebenen Teilstrahlengangs 28a, 28b erfasst. Die Synchronisations- und Steuereinrichtung 38 (oder eine hiervon separate Einheit) bildet aus den von dem Bildsensor 16 erfassten Bilddaten entsprechende Ströme von Paaren von Halbbildern und gibt diese letztlich an mehreren Datenausgängen 40a, 40b, 40c des Abbildungssystems 14 aus.

Insbesondere kann an einen Ausgang 40b des Abbildungssystems 14 eine Aufzeichnungseinrichtung 46 zum Aufzeichnen der erfassten Halbbilder angeschlossen werden. Ein Ausgang 40c des Abbildungssystems 14 kann mit einer Datenübertragungsschnittstelle 48 zum Fernübertragen der erfassten Halbbilder elektronisch verbunden sein, oder der Ausgang 40c bildet unmittelbar eine derartige Datenübertragungsschnittstelle 48.

Das Abbildungssystem 10 umfasst weiterhin ein Wiedergabesystem 50, welches zum Empfang der Bilddaten mit dem Ausgang 40a des Abbildungssystems 14 elektronisch verbunden ist. Unter einer elektronischen Verbindung wird in diesem Zusammenhang insbesondere eine kabelgebundene elektrische Verbindung, eine optische Verbindung, beispielsweise über Lichtleiter, oder auch eine drahtlose Datenverbindung verstanden. Das Wiedergabesystem 50 weist zwei Monitore 52a, 52b zum Darstellen der erfassten Halbbilder auf. Jedem Monitor 52a, 52b ist ein Okular 54a, 54b zugeordnet, wobei der Monitor 52a zur Wiedergabe der gemäß dem Teilstrahlengang 28a erfassten Halbbilder und der Monitor 52b zur Wiedergabe der gemäß dem Teilstrahlengang 28b erfassten Halbbilder vorgesehen ist.

Die Synchronisations- und Steuereinrichtung 38 kann mehrere Ausgänge aufweisen, an denen weitere Wiedergabesysteme angeschlossen werden können.

Anstelle des oben stehend beschriebenen Wiedergabesystems 50 können alternativ oder zusätzlich auch Wiedergabesysteme in anderer Ausgestaltung vorgesehen sein.

So kann ein alternatives nicht dargestelltes Wiedergabesystem einen einzigen Monitor aufweisen, welcher alternierend die Halbbilder eines jeweiligen Teilstrahlengangs 28a, 28b darstellt und der von einem Benutzer mit Hilfe einer entsprechend synchronisierten Shutterbrille betrachtet werden muss, welche abwechselnd eines der beiden Augen des Benutzers zur Betrachtung des Monitors freigibt.

Gemäß einer Abwandlung des Wiedergabesystems 50 kann das Wiedergabesystem auch nur einen einzigen Monitor umfassen, wobei die dargestellten Halbbilder mit Hilfe einer Strahlteilungseinrichtung alternierend zu einem der beiden Okulare 54a, 54b gelenkt werden, wie es vorstehend bereits erläutert wurde.

Das Abbildungssystem 10 kann ferner eine Beleuchtungseinrichtung 56 umfassen, welche zwei Beleuchtungseinheiten 58a, 58b aufweist, welche dazu angepasst sind, das Objekt 12 unter verschiedenen Beleuchtungswinkeln zu beleuchten. Die Ansteuerung der Beleuchtungseinrichtung 56 erfolgt durch die Synchronisations-und Steuereinrichtung 38 synchron zur Stellung der Verschlussblende 32 bzw. synchron zur Ansteuerung des elektronischen Shutters des Bildsensors 16. Je nach gewünschter Beleuchtungscharakteristik kann die Beleuchtungseinheit 58a gleichzeitig mit der Freigabe des Teilstrahlengangs 28a und die Beleuchtungseinheit 58b gleichzeitig mit der Freigabe des Teilstrahlengangs 28b aktiviert werden. Alternativ kann jedoch auch die Beleuchtungseinheit 58b gleichzeitig mit der Freigabe des Teilstrahlengangs 28a und die Beleuchtungseinheit 58a gleichzeitig mit der Freigabe des Teilstrahlengangs 28b aktiviert werden.

In einer nicht dargestellten Abwandlung kann die Beleuchtungseinrichtung auch mehrere Beleuchtungseinheiten umfassen, die das Objekt 12 jeweils mit einem unterschiedlichen Spektralbereich beleuchten können. So kann beispielsweise eine Beleuchtungseinheit sichtbares Licht emittieren, während eine weitere Beleuchtungseinheit beispielsweise zur Anregung eines mit fluoreszierenden Markerstoffen markierten Objekts 12 Licht im ultravioletten Spektralbereich emittieren kann. Die Ansteuerung der Beleuchtungseinheiten kann derart erfolgen, dass intermittierend ein erstes Paar von Halbbildern bei einer Beleuchtung mit sichtbarem Licht und ein zweites Paar von Halbbildern bei einer Beleuchtung mit ultraviolettem Licht erfasst wird. Im ersten Paar von Halbbildern ist das Objekt demnach im visuellen Spektralbereich sichtbar, während im zweiten Paar von Halbbildern im Wesentlichen nur die fluoreszierenden Marker erkennbar sind. Alternativ oder zusätzlich zu der weiteren Beleuchtungseinheit für einen ultravioletten Spektralbereich kann eine Beleuchtungseinheit für einen infraroten Spektralbereich vorgesehen sein. Bei diesen Ausführungsformen ist der Bildsensor 16 vorzugsweise für das gesamte Beleuchtungsspektrum empfindlich. Ferner kann eine breitbandig emittierende Beleuchtungseinrichtung mit einer Filtereinrichtung im Abbildungstrahlengang oder mit einem selektiv spektral empfindlichen Bildsensor kombiniert werden.

Mit Hilfe eines mit einer entsprechenden Auswahleinrichtung versehenen Wiedergabesystems können wahlweise nur die ersten Halbbilderpaare, nur die zweiten Halbbilderpaare oder eine elektronische Überlagerung der ersten und zweiten Halbbilderpaare dargestellt werden. Diese Darstellung erfolgt als Bewegtbilder, entsprechend den genannten Strömen von Halbbildern.

Grundsätzlich ist es auch möglich, zusätzlich weitere Spektralbereiche, beispielsweise den genannten infraroten Spektralbereich, mit einzubeziehen, wobei jeder Spektralbereich einzeln oder in beliebiger Kombination mit anderen Spektralbereichen wiedergegeben werden kann. Durch eine entsprechende Steuerung der Beleuchtungsstärke und/oder der Belichtungszeiten und/oder der Intensitätsverhältnisse bei der elektronischen Überlagerung der jeweiligen Halbbilder bei der Wiedergabe kann die Darstellung variiert werden. So kann beispielsweise der Anteil des visuellen Bereichs 50%, der Anteil des ultravioletten Bereichs 25% und der Anteil des Infrarotbereichs ebenfalls 25% betragen.

Mit Hilfe des stereoskopischen Abbildungssystems 10 können die mit Hilfe des Aufnahmesystems 14 erzeugten Halbbilder ohne Beeinträchtigung der Lichtintensität vervielfältigt und an eine beliebige Anzahl von Wiedergabesystemen 50, an eine oder mehrere Aufzeichnungseinrichtungen 46 und an eine oder mehrere Datenübertragungsschnittstellen 48 zur Fernübertragung der erzeugten Bilddaten angeschlossen werden. Das Wiedergabesystem 50 ist lediglich elektronisch mit dem Aufnahmesystem 14 verbunden, so dass eine Übertragung von mechanischen Stößen zwischen dem Aufnahmesystem 14 und dem Wiedergabesystem 50 weitgehend ausgeschlossen ist.

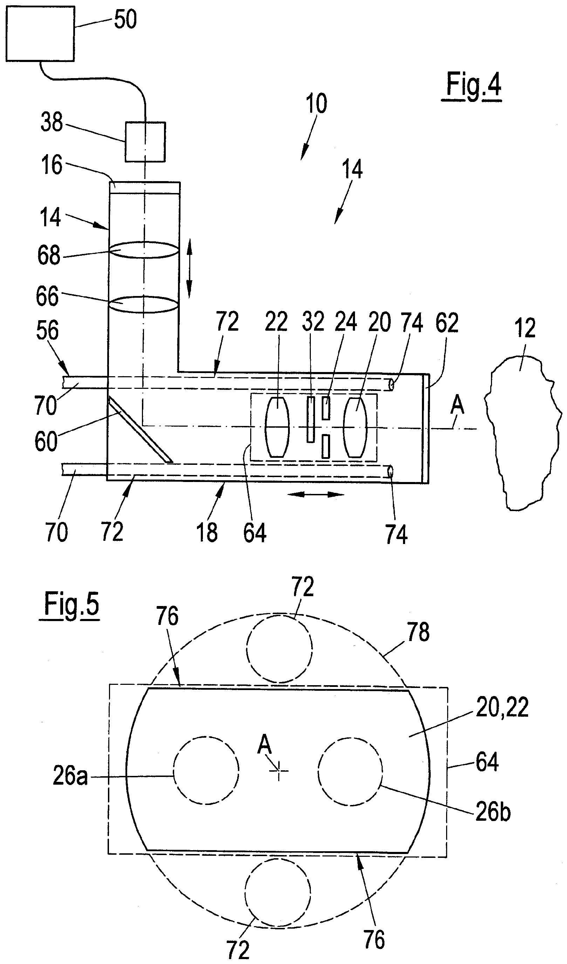

Fig. 4 zeigt in einer schematischen Seitenansicht eine weitere Ausführungsform eines stereoskopischen Abbildungssystems 10 zur Erzeugung von Paaren von stereoskopischen Halbbildern eines Objekts 12. Auch das Abbildungssystem 10 gemäß Fig. 4 umfasst ein Aufnahmesystem 14 mit einem elektronischen Bildsensor 16 und einem Objektiv 18, wobei das Objektiv 18 hier L-förmig ist. Ein Umlenkspiegel 60 lenkt den Abbildungsstrahlengang um 90° um.

Der dem abzubildenden Objekt 12 zugewandte Schenkel der L-Form ist in Richtung des Objekts 12 durch ein transparentes Verschlussfenster 62 geschlossen, das beispielsweise durch eine planparallele Glasplatte gebildet ist und als Schutz gegen Verschmutzung dient. Ausgehend von dem Verschlussfenster 62 folgen entlang der zentralen optischen Achse A als optische Elemente eine erste Linse 20 und eine zweite Linse 22, die gemeinsam mit einem zugeordneten Träger (nicht gezeigt) eine Linsengruppe 64 bilden. Die Linsengruppe 64 kann entlang der optischen Achse A als eine Einheit bewegt werden, um den Vergrößerungsfaktor des Objektivs 18 zu variieren (Zoom-Funktion). Zwischen der ersten Linse 20 und der zweiten Linse 22 sind eine innerhalb der Linsengruppe 64 feststehende Doppelaperturblende 24 und eine um die optische Achse A rotierende Verschlussblende 32 angeordnet. Die Doppelaperturblende 24 weist zwei voneinander beabstandete und zu der optischen Achse A versetzte Aperturen auf, die den Abbildungsstrahlengang in zwei Teilstrahlengänge für eine Rechts-Perspektive und eine Links-Perspektive unterteilen, wie im Zusammenhang mit Fig. 1 bis 3 erläutert.

In dem dem Bildsensor 16 zugewandten Schenkel des L-förmigen Objektivs 18 sind eine feststehende Fokussierlinse 66 und eine entlang der optischen Achse A bewegliche weitere Fokussierlinse 68 vorgesehen.

Mit dem Bildsensor 16 ist eine Synchronisations- und Steuereinrichtung 38 gekoppelt, welche auch mit einer Antriebsvorrichtung für die rotierende Verschlussblende 32 und einem zugeordneten Positionssensor verbunden ist (in Fig. 4 nicht gezeigt). Die Synchronisations- und Steuereinrichtung 38 synchronisiert die Bildfrequenz des Bildsensors 16 und die Drehzahl der rotierenden Verschlussblende 32, beispielsweise durch entsprechendes Ansteuern der Antriebsvorrichtung der Verschlussblende 32, wie ebenfalls bereits im Zusammenhang mit Fig. 1 bis 3 erläutert worden ist. Die Synchronisations- und Steuereinrichtung 38 bildet aus den von dem Bildsensor 16 erfassten Bilddaten entsprechende Ströme von Paaren von Halbbildern (entsprechend den beiden genannten Teilstrahlengängen) und gibt diese beispielsweise an ein elektronisches Wiedergabesystem 50 aus.

Das Abbildungssystem 10 gemäß Fig. 4 weist ferner eine Beleuchtungseinrichtung 56 auf, um das Objekt 12 mit sichtbarer Strahlung (d.h. Licht) und/oder unsichtbarer Strahlung (z.B. infraroter oder ultravioletter Strahlung) zu beleuchten. Die Beleuchtungseinrichtung 56 gemäß Fig. 4 umfasst zwei Lichtleiter 70, die an eine gemeinsame Lichtquelle (nicht gezeigt) angeschlossen sind. Jeder der beiden Lichtleiter 70 emittiert (insbesondere mittels einer Kollimationsoptik, nicht gezeigt) ein zumindest im Wesentlichen kollimiertes Beleuchtungsstrahlbündel 72. Das jeweilige Beleuchtungsstrahlbündel 72 verläuft innerhalb des betreffenden Schenkels des Objektivs 18 parallel zu der optischen Achse A, und zwar nach oben oder unten versetzt hierzu. Jedes der beiden Beleuchtungsstrahlbündel 72 beaufschlagt eine jeweilige Zerstreuungslinse 74, die das Beleuchtungsstrahlbündel 72 aufweitet und durch das Verschlussfenster 62 hindurch in Richtung des Objekts 12 umlenkt. Die beiden Zerstreuungslinsen 74 können innerhalb des Objektivs 18 feststehend angeordnet sein. Alternativ können die Zerstreuungslinsen 74 jedoch auch an dem genannten Träger der beweglichen Linsengruppe 64 befestigt sein und somit gemeinsam mit der Linsengruppe 64 bewegt werden.

Bei dem Abbildungssystem 10 gemäß Fig. 4 umfassen die beiden Linsen 20, 22 der Linsengruppe 64 des Objektivs 18 zwei Ausnehmungen, die es den genannten Beleuchtungsstrahlbündeln 72 ermöglichen, die Linsengruppe 64 zu passieren, ohne eine optische Grenzfläche der Linsengruppe 64 zu beaufschlagen. Somit wird ein durch die Beleuchtungsstrahlbündel 72 verursachter unerwünschter Streulichteinfall in die Linsengruppe 64 zuverlässig vermieden. Gleichwohl können die Beleuchtungsstrahlbündel 72 nahe der zentralen optischen Achse A bis zu den nahe der zentralen optischen Achse A angeordneten Zerstreuungslinsen 74 geführt werden, so dass eine Beleuchtung des Objekts 12 mit geringem Schattenwurf bewirkt wird und das Objektiv 18 einschließlich der teilweise integrierten Beleuchtungseinrichtung 56 kompakt baut (geringe radiale Ausdehnung bezüglich der optischen Achse A). Dies wird im Folgenden anhand der Fig. 5 näher erläutert.

Fig. 5 zeigt eine Frontansicht der beiden Linsen 20, 22 der Linsengruppe 64. Die Linsen 20, 22 weisen an der Oberseite und der Unterseite eine jeweilige Ausnehmung 76 in Form einer Abflachung auf. Mit anderen Worten besitzen die beiden Linsen 20, 22 nicht wie üblich einen geschlossenen kreisrunden Querschnitt 78 (wie in Fig. 5 gestrichelt gezeigt). Sondern ausgehend von einem derartigen kreisrunden Querschnitt 78 sind an zwei zueinander parallel und in der Frontansicht gemäß Fig. 5 horizontal verlaufenden Sekanten der Kreisform die zwei Ausnehmungen 76 vorgesehen, die sich entlang der Linsengruppe 64 parallel zu der optischen Achse A erstrecken.

Die Linsen 20, 22 müssen allerdings nicht, wie in Fig. 5 gezeigt, geradlinig "abgeschnitten" sein. Beispielsweise könnten die Ausnehmungen 76 auch durch kreisrunde Bohrungen innerhalb eines kreisrunden Querschnitt 78 der Linsen 20, 22 gebildet sein. Wichtig ist lediglich, dass die Ausnehmungen 76 eine ungehinderte Ausbreitung des jeweiligen Beleuchtungsstrahlbündels 72 ermöglichen, obwohl die beiden Beleuchtungsstrahlbündel 72 innerhalb des Objektivs 18 verlaufen.

Zum besseren Verständnis sind in Fig. 5 auch noch die beiden Aperturen 26a, 26b der Doppelaperturblende 24 gezeigt, welche in Flucht zu den beiden Linsen 20, 22 angeordnet ist (gestrichelte Darstellung, ohne Berücksichtigung der durch die Linsen 20, 22 bewirkte Vergrößerung der Darstellung). Die Ausnehmungen 76 sind sowohl bezüglich der optischen Achse A als auch bezüglich der beiden Aperturen 26a, 26b versetzt. Aus Fig. 5 ist somit auch ersichtlich, dass die Ausnehmungen 76, bezogen auf eine Betrachtung entlang der optischen Achse A, einen gewissen Mindestabstand zu den Aperturen 26a, 26b der Doppelaperturblende 24 einhalten.

Mit neuerlicher Bezugnahme auf Fig. 4 sei noch darauf hingewiesen, dass die beiden Zerstreuungslinsen 74 bezogen auf die Ausbreitungsrichtung der Beleuchtungsstrahlbündel 72 jenseits der ersten Linse 20 angeordnet sind. Mit anderen Worten sind die beiden Zerstreuungslinsen 74 aus der Sicht des Bildsensors 16 hinter dem letzten (d.h. Objekt-nächsten) optischen Element des Objektivs 18 angeordnet. Hierdurch wird erreicht, dass trotz des Aufweitens der Beleuchtungsstrahlbündel 72 keine Lichtbeaufschlagung eines optisch wirksamen Elements des Objektivs 18 erfolgt, was zu unerwünschten Lichtstreueffekten führen könnte.

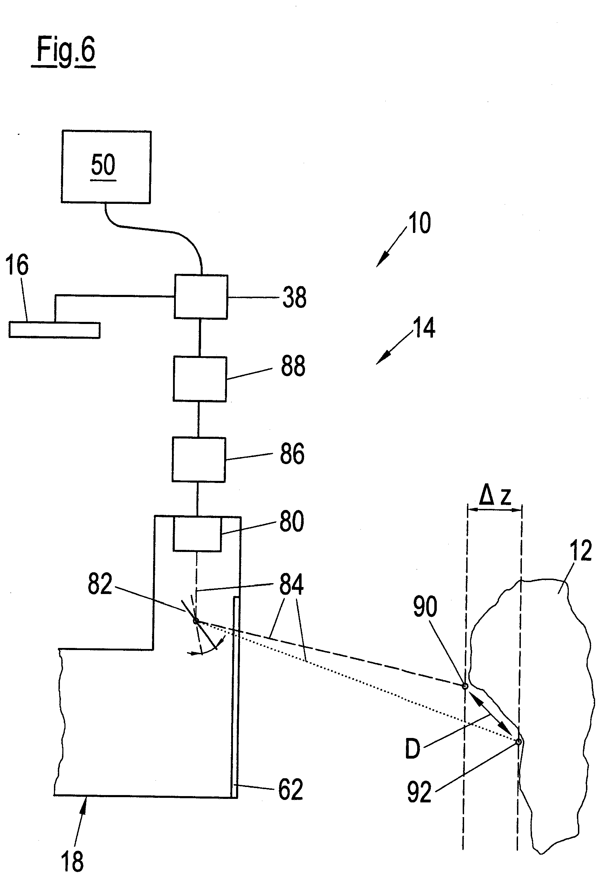

Fig. 6 zeigt in einer schematischen Seitenansicht das dem abzubildenden Objekt 12 zugewandte Ende eines Objektivs 18 eines stereoskopischen Abbildungssystems 10, beispielsweise gemäß Fig. 1 bis 3 oder gemäß Fig. 4 und 5, wobei das Abbildungssystem 10 gemäß Fig. 6 die Bestimmung eines tiefenabhängigen Abstands zwischen zwei Objektmarkierungspunkten an dem Objekt 12 gestattet. Hierzu ermöglicht das Abbildungssystem 10 gemäß Fig. 6 eine besonders gut visuell erfassbare und elektronisch diskriminierbare Markierung des Objekts 12 mittels eines Lichtflecks, der hier durch einen Laser-Spot gebildet ist.

Das Aufnahmesystem 14 des Abbildungssystems 10 gemäß Fig. 6 weist hierfür eine Markierungsstrahl-Sendeeinrichtung auf, die eine Laserstrahl-Lichtquelle 80 und einen beweglichen Umlenkspiegel 82 umfasst. Die Laserstrahl-Lichtquelle 80 vermag einen Markierungslichtstrahl 84 zu emittieren, der von dem Umlenkspiegel 82 in variabler Richtung umgelenkt wird (vgl. gestrichelte und gepunktete Darstellung in Fig. 6), um an dem Objekt 12 an einer auswählbaren Position einen Lichtfleck zu erzeugen.

Damit ein Benutzer (insbesondere der Operateur) die Position des Lichtflecks auswählen kann, umfasst das Abbildungssystem 10 gemäß Fig. 6 ferner eine Eingabeeinrichtung 86 (z.B. Steuerknüppel mit Taste). Die Eingabeeinrichtung 86 ist direkt oder über eine zugeordnete Steuereinrichtung mit einer Antriebseinrichtung des beweglichen Umlenkspiegels 82 gekoppelt (in Fig. 6 nicht gezeigt). Die Eingabeeinrichtung 86 ist ferner mit einer Auswerte- und Steuereinrichtung 88 gekoppelt, die mit einer Synchronisations- und Steuereinrichtung 38 entsprechend Fig. 1 oder Fig. 4 gekoppelt ist. Die Synchronisations- und Steuereinrichtung 38 wiederum ist eingangsseitig ferner mit einem Bildsensor 16 des Abbildungssystems 10 und ausgangsseitig mit einem Wiedergabesystem 50 gekoppelt. Die Auswerte- und Steuereinrichtung 88 kann alternativ auch eine Untereinheit der Synchronisations- und Steuereinrichtung 38 sein.

Wenn die Eingabeeinrichtung 86 einen Richtungsänderungsbefehl des Benutzers erfasst (z.B. Verschwenken eines Steuerknüppels), erzeugt die Eingabeeinrichtung 86 ein entsprechendes Richtungssteuersignal, um durch Verkippen des Umlenkspiegels 82 die Ausrichtung des Markierungslichtstrahls 84 zu ändern. Der Benutzer kann gleichzeitig unter stereoskopischer Betrachtung den an dem Objekt 12 entsprechend erzeugten Lichtfleck an dem Wiedergabesystem 50 beobachten, das entsprechend der Ausführungsform gemäß Fig. 1 bis 3 oder gemäß Fig. 4 und 5 ein jeweiliges Paar von Halbbildern wiedergibt, die von dem Bildsensor 16 des Abbildungssystems 10 erfasst werden. Wenn der Benutzer die Ausrichtung des Markierungslichtstrahls 84 ändert, kann er dies durch ein Wandern des erzeugten Lichtflecks entlang des abgebildeten Objekts 12 beobachten. Damit eine derartige Beobachtung des Lichtflecks an dem Wiedergabesystem 50 möglich ist, muss der Spektralbereich der an dem Wiedergabesystem 50 wiedergegebenen Halbbilder natürlich die Wellenlänge des Markierungslichtstrahls 84 umfassen.

Sobald die Eingabeeinrichtung 86 einen Markierungspunktauswahlbefehl des Benutzers erfasst (z.B. Drücken einer Taste), erzeugt die Eingabeeinrichtung 86 ein entsprechendes Markierungspunktauswahlsignal, um der Auswerte- und Steuereinrichtung 88 anzuzeigen, dass die momentane Position des Lichtflecks an dem Objekt 12 einen ersten oder zweiten ausgewählten Objektmarkierungspunkt kennzeichnen soll. In Fig. 6 sind ein derartiger erster Objektmarkierungspunkt 90 und ein zweiter Objektmarkierungspunkt 92 gezeigt. Die Auswerte- und Steuereinrichtung 88 ordnet die jeweilige momentane Position des Lichtflecks an dem Objekt 12, die dem ersten oder zweiten Objektmarkierungspunkt 90, 92 entspricht, einer jeweiligen Bildpunktposition innerhalb der beiden Bilddatensätze zu, die den beiden am Wiedergabesystem 50 wiedergegebenen Halbbildern entsprechen. Hierdurch wird also innerhalb der beiden Bilddatensätze ein jeweiliger Bildmarkierungspunkt festgelegt. Hierfür kann die Auswerte- und Steuereinrichtung 88 die Position des wiedergegebenen Lichtflecks innerhalb des jeweiligen Halbbildes durch ein bekanntes Verfahren der Bildanalyse identifizieren (z.B. durch eine Kontrastanalyse) und die identifizierte Position einem Bildpunkt innerhalb des Bilddatensatzes des jeweiligen Halbbildes zuordnen. Diese jeweilige Bildpunktposition ist für die beiden Halbbilder eines Paares nicht unbedingt identisch, da die beiden Halbbilder zwei verschiedenen stereoskopischen Perspektiven entsprechen.

Die genannte Identifizierung der Position des Lichtflecks innerhalb des jeweiligen Halbbild-Datensatzes ist bei der Ausführungsform gemäß Fig. 6 besonders einfach, da es aufgrund der Beaufschlagung des Objekts 12 mit dem Markierungslichtstrahl 84 möglich ist, einen Lichtfleck an dem Objekt 12 zu erzeugen, der einen besonders deutlichen Kontrast (Helligkeit und/oder Wellenlänge) zu seiner Umgebung bildet.

Aufgrund der getroffenen Zuordnung sorgt die Auswerte- und Steuereinrichtung 88 ferner dafür, dass die den beiden Objektmarkierungspunkten 90, 92 entsprechenden Bildmarkierungspunkte an dem Wiedergabesystem 50 für den Benutzer wahrnehmbar auf den wiedergegebenen Halbbildern dargestellt werden (vorzugsweise in einer anderen Farbe als die inhärente Wiedergabe des Lichtflecks). Hierfür kann die Auswerte- und Steuereinrichtung 88 beispielsweise mit einem elektronischen Bildgenerator gekoppelt sein (nicht gezeigt), um den an dem Wiedergabesystem 50 wiedergegebenen Halbbildern die Bildmarkierungspunkte zu überlagern. Beispielsweise können die Bildpunkte an den genannten identifizierten Positionen innerhalb des Bilddatensatzes des jeweiligen Halbbildes eine andere Farbe zugeordnet erhalten, oder die Bildmarkierungspunkte werden durch graphische Symbole (z.B. Kreuz) angezeigt.

Nachdem die beiden Objektmarkierungspunkte 90, 92 ausgewählt worden sind, berechnet die Auswerte- und Steuereinrichtung 88 einen tiefenabhängigen Abstand D zwischen dem ersten Objektmarkierungspunkt 90 und dem zweiten Objektmarkierungspunkt 92, d.h. die Auswerte- und Steuereinrichtung 88 berücksichtigt nicht nur die Ausrichtung einer Verbindungslinie zwischen den beiden Objektmarkierungspunkten 90, 92 innerhalb einer Normalebene zu der optischen Achse A, sondern auch eine Erstreckung der genannten Verbindungslinie entlang der optischen Achse A (z-Koordinate). Diese Erstreckung entlang der optischen Achse A entspricht einem Tiefenunterschied Δz des ersten Objektmarkierungspunkts 90 und des zweiten Objektmarkierungspunkts 92. Um die Tiefenposition des jeweiligen Objektmarkierungspunkts 90, 90 zu ermitteln, kann die Auswerte- und Steuereinrichtung 88 beispielsweise die Disparität der beiden Halbbilder des betreffenden Paares analysieren oder für unterschiedlich eingestellte Bildweiten des Objektivs 18 die jeweilige Modulationsübertragungsfunktion im Bereich der (den beiden Objektmarkierungspunkten 90, 92 entsprechenden) Bildmarkierungspunkte analysieren. Wenn auf diese Weise die Position der beiden Objektmarkierungspunkte 90, 92 im Raum ermittelt worden ist (d.h. wenn die Koordinaten der beiden Objektmarkierungspunkten 90, 92 beispielsweise in einem kartesischen Koordinatensystem vollständig bekannt sind), ergibt sich der Abstand D aufgrund einfacher vektorgeometrischer Beziehungen.

Indem also der Benutzer (z.B. Operateur) unter Zuhilfenahme der Eingabeeinrichtung 86 nacheinander zwei Objektmarkierungspunkte 90, 92 auswählt, die an dem Wiedergabesystem zunächst als Lichtfleck und nach erfolgter Auswahl als entsprechende Markierungspunktsymbole beobachtet werden können, kann der Benutzer eine geradlinige, im Raum beliebig ausgerichtete Vermessungsstrecke definieren, zu der die Auswerte- und Steuereinrichtung 88 die Länge berechnet. Der berechnete Wert kann sodann beispielsweise an dem Wiedergabesystem 50 angezeigt werden.

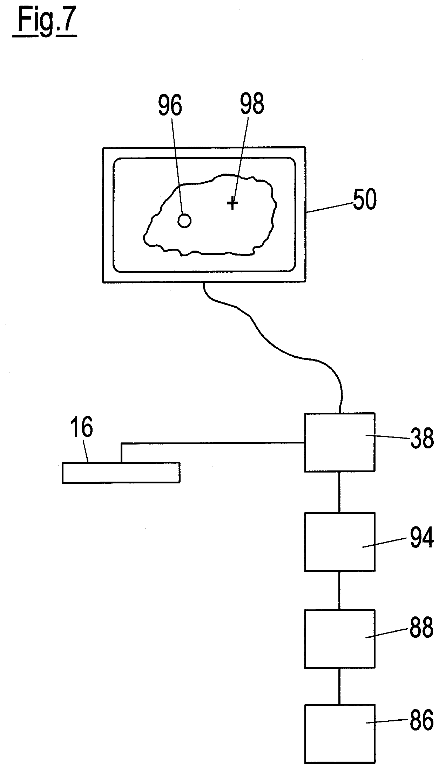

Fig. 7 illustriert anhand eines Blockschaltbilds, dass eine Markierungsstrahl-Sendeeinrichtung (Laserstrahl-Lichtquelle 80 und Umlenkspiegel 82) gemäß Fig. 6 nicht zwingend erforderlich ist, um eine elektronische tiefenabhängige In-Bild-Abstandsmessung bei einem stereoskopischen Abbildungssystem 10 durchzuführen. Gezeigt sind lediglich die in diesem Zusammenhang relevanten Komponenten des Abbildungssystems 10, das im Übrigen beispielsweise wie bei der Ausführungsform gemäß Fig. 1 bis 3 oder gemäß Fig. 4 und 5 aufgebaut sein kann. Das Abbildungssystem 10 gemäß Fig. 7 umfasst ähnlich der Ausführungsform gemäß Fig. 6 einen Bildsensor 16, der wenigstens einen Strom von Paaren von Halbbildern über eine Synchronisations- und Steuereinrichtung 38 an ein Wiedergabesystem 50 liefert. Gezeigt sind ferner eine Eingabeeinrichtung 86 und eine hiermit gekoppelte Auswerte- und Steuereinrichtung 88. Die Auswerte- und Steuereinrichtung 88 ist mit einem Bildgenerator 94 gekoppelt, der wiederum mit der Synchronisations- und Steuereinrichtung 38 gekoppelt ist. Die Synchronisations- und Steuereinrichtung 38, die Auswerte- und Steuereinrichtung 88 und der Bildgenerator 94 können auch durch eine einzige Einheit gebildet sein.

An dem Wiedergabesystem 50 wird ein jeweiliges Paar von Halbbildern wiedergebeben, die von dem Bildsensor 16 erfasst worden sind. Der Bildgenerator 94 wird von der Auswerte- und Steuereinrichtung 88 dazu angesteuert, den wiedergegebenen Halbbildern ein jeweiliges Positionsanzeigesymbol 96 (z.B. Kreis-Symbol) und/oder ein jeweiliges Markierungspunktsymbol 98 (z.B. Kreuz-Symbol) zu überlagern. Wenn der Benutzer (z.B. Operateur) geeignete Positionseingabebefehle an der Eingabeeinrichtung 86 eingibt (z.B. Überstreichen einer berührungsempfindlichen Oberfläche des Wiedergabesystems 50), veranlasst die Auswerte- und Steuereinrichtung 88 den Bildgenerator 94 dazu, die Darstellung des jeweiligen Positionsanzeigesymbols 96 entsprechend zu versetzen. Wenn der Benutzer einen Markierungspunktauswahlbefehl an der Eingabeeinrichtung 86 eingibt (z.B. Antippen der berührungsempfindlichen Oberfläche des Wiedergabesystems 50 an der Stelle des dargestellten Positionsanzeigesymbols 96), interpretiert die Auswerte- und Steuereinrichtung 88 dies als Eingabe eines gewünschten Bildmarkierungspunkts. Die Auswerte- und Steuereinrichtung 88 veranlasst den Bildgenerator 94 nun dazu, die Darstellung des jeweiligen Positionsanzeigesymbols 96 in eine Darstellung eines jeweiligen Markierungspunktsymbols 98 zu ändern. Ferner ordnet die Auswerte- und Steuereinrichtung 88 die Position dieses neu dargestellten Markierungspunktsymbols 98 einer jeweiligen Bildpunktposition innerhalb der Bilddatensätze zu, die den an dem Wiedergabesystem 50 wiedergegebenen Halbbildern entsprechen.

Hierdurch kann der Benutzer auf einfache Weise einen ersten Bildmarkierungspunkt und einen zweiten Bildmarkierungspunkt definieren, die der gewünschten Abstandsberechnung zugrunde gelegt werden sollen. Die Auswerte- und Steuereinrichtung 88 bestimmt sodann zu dem ersten Bildmarkierungspunkt und dem zweiten Bildmarkierungspunkt eine jeweilige Tiefenposition, um die Lage entsprechender Objektmarkierungspunkte an dem abgebildeten Objekt, d.h. an dem tatsächlichen räumlichen Objekt zu ermitteln. Hieraus berechnet die Auswerte- und Steuereinrichtung 88 den tiefenabhängigen Abstand zwischen beiden ermittelten Objektmarkierungspunkten, wie im Zusammenhang mit Fig. 6 erläutert.

### Bezugszeichenliste

- 10: Abbildungssystem
- 12: Objekt
- 14: Aufnahmesystem
- 16: Bildsensor
- 18: Objektiv
- 20: erste Linse
- 22: zweite Linse
- 24: Doppelaperturblende
- 26a, 26b: Apertur
- 28a, 28b: Teilstrahlengang
- 30: Antriebsvorrichtung
- 32: Verschlussblende
- 34: Segmentöffnung
- 36: Positionssensor
- 38: Synchronisations- und Steuereinrichtung
- 40a, 40b, 40c: Ausgang des Abbildungssystems
- 42: Sensorausgang
- 44: Sensoreingang
- 46: Aufzeichnungseinrichtung
- 48: Datenübertragungsschnittstelle
- 50: Wiedergabesystem
- 52a, 52b: Monitor
- 54a, 54b: Okular
- 56: Beleuchtungseinrichtung
- 58a, 58b: Beleuchtungseinheit
- 60: Umlenkspiegel
- 62: Verschlussfenster
- 64: Linsengruppe
- 66: Fokussierlinse
- 68: Fokussierlinse
- 70: Lichtleiter
- 72: Beleuchtungsstrahlbündel
- 74: Zerstreuungslinse
- 76: Ausnehmung
- 78: kreisrunder Querschnitt
- 80: Laserstrahl-Lichtquelle
- 82: Umlenkspiegel
- 84: Markierungslichtstrahl
- 86: Eingabeeinrichtung
- 88: Auswerte- und Steuereinrichtung
- 90: erster Objektmarkierungspunkt
- 92: zweiter Objektmarkierungspunkt
- 94: Bildgenerator
- 96: Positionsanzeigesymbol
- 98: Markierungspunktsymbol

- A: optische Achse
- Δz: Tiefenunterschied

## Patentansprüche

1. Stereoskopisches Abbildungssystem in Form eines Mikroskops zur Erzeugung von Paaren von stereoskopischen Halbbildern, umfassend ein Aufnahmesystem (14) mit:
- einem einzigen elektronischen Bildsensor (16), welcher einen elektronischen Shutter zum Einstellen einer Ladungsintegrationszeit des Bildsensors (16) aufweist;
- einem Objektiv (18) zum Erzeugen eines Abbildes eines Objekts (12) auf dem Bildsensor, wobei das Objektiv (18) einen einzigen Abbildungsstrahlengang mit einer zentralen optischen Achse (A) aufweist;
- einer im Abbildungsstrahlengang angeordneten Doppelaperturblende (24), welche zwei voneinander beabstandete und zu der optischen Achse (A) versetzte Aperturen (26a, 26b) aufweist, die den Abbildungsstrahlengang in jeweilige Teilstrahlengänge (28a, 28b) unterteilen, wobei sich die Teilstrahlengänge (28a, 28b) hinsichtlich ihres Betrachtungswinkels des Objekts (12) unterscheiden;
- einer der Doppelaperturblende (24) zugeordneten rotierenden Verschlussblende (32), welche gemäß einer Drehzahl zeitlich alternierend eine der beiden Aperturen (26a, 26b) für eine vorbestimmte Zeitperiode freigibt und die andere der beiden Aperturen (26a, 26b) zumindest während dieser Zeitperiode verschließt, um zeitlich alternierend einen der beiden Teilstrahlengänge (28a, 28b) freizugeben und den anderen der beiden Teilstrahlengänge (28a, 28b) zu unterbrechen; und
- einer Synchronisations- und Steuereinrichtung (38), welche dazu angepasst ist, die durch den elektronischen Shutter des Bildsensors (16) eingestellte Ladungsintegrationszeit und die Drehzahl der rotierenden Verschlussblende (32) derart miteinander zu synchronisieren, dass der Bildsensor (16) während der genannten Zeitperiode ein jeweiliges stereoskopisches Halbbild auf der Grundlage des freigegebenen Teilstrahlengangs (28a, 28b) erfasst;
**dadurch gekennzeichnet,**
**dass** das Abbildungssystem (10) dazu angepasst ist, einen von wenigstens zwei vorbestimmten Spektralbereichen auszuwählen, welcher von dem Bildsensor (16) zum Erfassen eines jeweiligen Paares von stereoskopischen Halbbildern berücksichtigt wird, wobei das Abbildungssystem (10) ferner dazu angepasst ist, den Spektralbereich zeitlich alternierend auszuwählen und die Paare von stereoskopischen Halbbildern in Abhängigkeit von dem ausgewählten Spektralbereich einer entsprechenden Bildgruppe zuzuordnen, so dass jeder Bildgruppe nur diejenigen Halbbilder zugeordnet sind, die demselben ausgewählten Spektralbereich entsprechen.

2. Abbildungssystem nach Anspruch 1,
wobei die Synchronisations- und Steuereinrichtung (38) dazu angepasst ist, die Drehzahl der rotierenden Verschlussblende (32) an eine Bildfrequenz des Bildsensors (16) anzupassen, die der eingestellten Ladungsintegrationszeit entspricht; oder
wobei die Synchronisations- und Steuereinrichtung (38) dazu angepasst ist, eine Bildfrequenz des Bildsensors (16), die der eingestellten Ladungsintegrationszeit entspricht, an eine vorgegebene Drehzahl der rotierenden Verschlussblende (32) anzupassen.

3. Abbildungssystem nach Anspruch 1 oder 2,
wobei das Abbildungssystem (10) ferner eine Beleuchtungseinrichtung (56) umfasst, welche dazu angepasst ist, das Objekt (12) alternierend aus zwei unterschiedlichen Beleuchtungswinkeln zu beleuchten, wobei jedem Beleuchtungswinkel einer der Teilstrahlengänge (28a, 28b) zugeordnet ist, und wobei die Synchronisations- und Steuereinrichtung (38) ferner dazu angepasst ist, den jeweiligen Beleuchtungswinkel synchron zu dem Freigeben des zugeordneten Teilstrahlengangs (28a, 28b) zu aktivieren.

4. Abbildungssystem nach Anspruch 1 oder 2,
wobei das Abbildungssystem (10) ferner eine Beleuchtungseinrichtung (56) umfasst, welche dazu angepasst ist, wenigstens ein Beleuchtungsstrahlbündel (72) zum Beleuchten des Objekts (12) zu erzeugen, wobei wenigstens eine Linse (20, 22) des Objektivs (18) wenigstens eine Ausnehmung (76) aufweist, durch die der Querschnitt der genannten wenigstens einen Linse (20, 22) sich von der Form einer geschlossenen Kreisfläche (78) unterscheidet, und wobei das wenigstens eine Beleuchtungsstrahlbündel (72) entlang der optischen Achse (A) innerhalb der Ausnehmung (76) verläuft.

5. Abbildungssystem nach Anspruch 4,
wobei die wenigstens eine Ausnehmung (76) entlang der optischen Achse (A) betrachtet versetzt zu den zwei Aperturen (26a, 26b) der Doppelaperturblende (24) ist.

6. Abbildungssystem nach Anspruch 4 oder 5,
wobei die Beleuchtungseinrichtung (56) wenigstens eine Zerstreuungslinse (74) zum Aufweiten des wenigstens einen Beleuchtungsstrahlbündels (72) aufweist, wobei die wenigstens eine Zerstreuungslinse (74) bezogen auf die Ausbreitungsrichtung des wenigstens einen Beleuchtungsstrahlbündels (72) hinter der genannten wenigstens einen Linse (20, 22) angeordnet ist.

7. Abbildungssystem nach Anspruch 1 oder 2,
wobei das Abbildungssystem (10) ferner eine Beleuchtungseinrichtung zum Beleuchten des Objekts umfasst, wobei die Synchronisations- und Steuereinrichtung (38) dazu angepasst ist, die Beleuchtungseinrichtung alternierend zur Erzeugung einer unterschiedlichen Beleuchtungscharakteristik anzusteuern und den elektronischen Bildsensor (16) zum Erfassen der Halbbilder mit einer Bildfrequenz von wenigstens 50 Hz anzusteuern.

8. Abbildungssystem nach einem der vorhergehenden Ansprüche,
wobei das Abbildungssystem (10) wenigstens ein Wiedergabesystem (50) zum Wiedergeben der erfassten Halbbilder aufweist, das mit einem Ausgang des Aufnahmesystems (14) elektronisch verbunden ist.

9. Abbildungssystem nach Anspruch 8,
wobei das Wiedergabesystem (50) einen einzigen Monitor zum Darstellen der erfassten Halbbilder und ein Paar von Okularen (54a, 54b) aufweist, wobei das Wiedergabesystem (50) ferner eine Strahlteilungseinrichtung umfasst, welche die gemäß dem einen Teilstrahlengang (28a) erfassten Halbbilder zu dem einen Okular (54a) und die gemäß dem anderen Teilstrahlengang (28b) erfassten Halbbilder zu dem anderem Okular (54b) lenkt; oder
wobei das Wiedergabesystem (50) zwei Monitore (52a, 52b) zum Darstellen der erfassten Halbbilder aufweist, wobei jedem Monitor (52a) ein Okular zugeordnet ist, und wobei der eine Monitor zur Wiedergabe der gemäß dem einen Teilstrahlengang (28a) erfassten Halbbilder und der andere Monitor (52b) zur Wiedergabe der gemäß dem anderen Teilstrahlengang (28b) erfassten Halbbilder vorgesehen ist.

10. Abbildungssystem nach Anspruch 8 oder 9,
wobei das Abbildungssystem (10) ferner eine Eingabeeinrichtung (86) umfasst, die dazu angepasst ist, einen jeweiligen ersten Bildmarkierungspunkt und einen jeweiligen zweiten Bildmarkierungspunkt innerhalb der an dem Wiedergabesystem (50) wiedergegebenen Halbbilder festzulegen, wobei der jeweilige erste Bildmarkierungspunkt an dem Objekt (12) einem ersten Objektmarklerungspunkt (90) und der jeweilige zweite Bildmarkierungspunkt an dem Objekt (12) einem zweiten Objektmarkierungspunkt (92) entspricht, die entlang der optischen Achse (A) in unterschiedlicher Tiefe angeordnet sein können, und
wobei das Abbildungssystem (10) eine Auswerte- und Steuereinrichtung (88) umfasst, die dazu angepasst ist, einen tiefenabhängigen Abstand (D) zwischen dem ersten Objektmarkierungspunkt (90) und dem zweiten Objektmarkierungspunkt (92) zu berechnen.

11. Abbildungssystem nach einem der vorhergehenden Ansprüche,
wobei das Abbildungssystem (10) ferner wenigstens eine Aufzeichnungseinrichtung (46) zum Aufzeichnen der erfassten Halbbilder aufweist, die mit einem Ausgang des Bildsensors (16) des Aufnahmesystems (14) elektronisch verbunden ist.

12. Abbildungssystem nach einem der vorhergehenden Ansprüche,
wobei das Abbildungssystem (10) ferner wenigstens eine Datenübertragungsschnittstelle (48) zum Fernübertragen der erfassten Halbbilder aufweist, die mit einem Ausgang des Bildsensors (16) des Aufnahmesystems (14) elektronisch verbunden ist.

13. Abbildungssystem nach einem der vorhergehenden Ansprüche,
wobei das Aufnahmesystem (14) wenigstens einen ersten Datenausgang (40a) aufweist, der dazu angepasst ist, die erfassten Halbbilder an ein Wiedergabesystem (50) zum Wiedergeben der Halbbilder auszugeben, wobei das Aufnahmesystem (14) wenigstens einen zweiten Datenausgang (40b, 40c) aufweist, der dazu angepasst ist, die erfassten Halbbilder an eine Aufzeichnungseinrichtung (46) zum Aufzeichnen der Halbbilder auszugeben oder die erfassten Halbbilder an ein Datennetz (48) zum Fernübertragen der Halbbilder auszugeben, wobei das Abbildungssystem (10) ferner eine Beleuchtungseinrichtung zum Beleuchten des Objekts gemäß einer variablen Beleuchtungscharakteristik umfasst, wobei die Synchronisations- und Steuereinrichtung (38) dazu angepasst ist, den elektronischen Bildsensor (16) zum Erfassen der Halbbilder mit einer Bildfrequenz von wenigstens 50 Hz anzusteuern und die Beleuchtungseinrichtung synchron hierzu alternierend zur Erzeugung wenigstens einer ersten und einer zweiten Beleuchtungscharakteristik anzusteuern, und wobei die Synchronisations- und Steuereinrichtung (38) ferner dazu angepasst ist, an dem ersten Datenausgang (40a) wenigstens einen ersten Strom von Paaren von Halbbildern und an dem zweiten Datenausgang (40b, 40c) wenigstens einen zweiten Strom von Paaren von Halbbildern auszugeben, wobei die beiden Ströme von Paaren von Halbbildern sich hinsichtlich der für die Erzeugung der Halbbilder verwendeten jeweiligen Beleuchtungscharakteristik zumindest teilweise voneinander unterscheiden.

14. Abbildungssystem nach einem der vorhergehenden Ansprüche,
wobei das Abbildungssystem (10) ferner eine Auswahleinrichtung zur Auswahl wenigstens einer der genannten Bildgruppen aufweist, welche dazu angepasst ist, das Abbildungssystem (10) so zu steuern, dass nur die der ausgewählten Bildgruppe zugeordneten Halbbilder wiedergegeben werden.

15. Abbildungssystem nach einem der vorhergehenden Ansprüche,
wobei das Abbildungssystem (10) einen Positionssensor (36) aufweist, welcher der Verschlussblende (32) zugeordnet ist und dazu angepasst ist, die Winkelposition der Verschlussblende (32) zu ermitteln und der ermittelten Winkelposition entsprechende Positionssignale an die Synchronisations- und Steuereinrichtung (38) zu übermitteln, wobei die Positionssignale Informationen darüber umfassen, welcher der Teilstrahlengänge (28a, 28b) momentan freigegeben oder unterbrochen ist, und
wobei die Synchronisations- und Steuereinrichtung (38) ferner dazu angepasst ist, auf der Grundlage der übermittelten Positionssignale jeweilige Kanalkennungen derart zu erzeugen und mit den von dem Bildsensor (16) erfassten Halbbildern zu verknüpfen, dass jedes Halbbild anhand der hiermit verknüpften Kanalkennung dem für das Erfassen dieses Halbbildes freigegebenen Teilstrahlengang (28a, 28b) zugeordnet ist.

## Claims

1. A stereoscopic imaging system in the form of a microscope for generating pairs of stereoscopic half-images comprising a taking system (14) having:
- a single electronic image sensor (16) that has an electronic shutter for setting a charge integration time of the image sensor (16);
- an objective (18) for generating an image of an object (12) on the image sensor, wherein the objective (18) has a single imaging optical path having a central optical axis (A);
- a double aperture diaphragm (24) that is arranged in the imaging optical path and that has two mutually spaced apart apertures (26a, 26b) that are offset from the optical axis (A) and that divide the imaging optical path into respective part optical paths (28a, 28b), wherein the part optical paths (28a, 28b) differ with respect to their viewing angle of the object (12);
- a rotating shutter assembly (32) that is associated with the double aperture diaphragm (24), that uncovers one of the two apertures (26a, 26b) alternately in time for a predefined time period in accordance with a speed of rotation and that closes the other one of the two apertures (26a, 26b) at least during this time period in order to release one of the two part optical paths (28a, 28b) and to interrupt the other one of the two part optical paths (28a, 28b) alternately in time; and
- a synchronization and control device (38) that is adapted to synchronize the charge integration time set by the electronic shutter of the image sensor (16) and the speed of rotation of the rotating shutter assembly (32) with one another such that the image sensor (16) detects a respective stereoscopic half-image on the basis of the released part optical path (28a, 28b) during the named time period,
**characterized in that**
the imaging system (10) is adapted to select one of at least two predefined spectral ranges that is taken into account by the image sensor (16) for detecting a respective pair of stereoscopic half-images, with the imaging system (10) further being adapted to select the spectral range alternately in time and to associate the pairs of stereoscopic half-images to a corresponding image group in dependence on the selected spectral range such that only those half-images are associated with each image group that correspond to the same selected spectral range.

2. An imaging system in accordance with claim 1,
wherein the synchronization and control device (38) is adapted to match the speed of rotation of the rotating shutter assembly (32) to a frame rate of the image sensor (16) that corresponds to the set charge integration time; or wherein the synchronization and control device (38) is adapted to match a frame rate of the image sensor (16) that corresponds to the set charge integration time to a predefined speed of rotation of the rotating shutter assembly (32).

3. An imaging system in accordance with claim 1 or claim 2,
wherein the imaging system (10) furthermore comprises a lighting device (56) that is adapted to light the object (12) alternately from two different lighting angles; wherein one of the part optical paths (28a, 28b) is associated with each lighting angle; and wherein the synchronization and control device (38) is furthermore adapted to activate the respective lighting angle synchronously with the release of the associated part optical path (28a, 28b).

4. An imaging system in accordance with claim 1 or claim 2,
wherein the imaging system (10) furthermore comprises a lighting device (56) that is adapted to generate at least one lighting beam (27) for lighting the object (12); wherein at least one lens (20, 22) of the objective (18) has at least one cut-out (76) by which the cross-section of the named at least one lens (20, 22) differs from the shape of a closed circular area (78); and wherein the at least one lighting beam (72) extends along the optical axis (A) within the cut-out (76).

5. An imaging system in accordance with claim 4,
wherein the at least one cut-out (76) is offset from the two apertures (26a, 26b) of the double aperture diaphragm (24) viewed along the optical axis (A).

6. An imaging system in accordance with claim 4 or claim 5,
wherein the lighting device (56) has at least one diverging lens (74) for expanding the at least one lighting beam (72), with the at least one diverging lens (74) being arranged behind the named at least one lens (20, 22) with respect to the propagation direction of the at least one lighting beam (72).

7. An imaging system in accordance with claim 1 or claim 2,
wherein the imaging system (10) furthermore comprises a lighting device for lighting the object, with the synchronization and control device (38) being adapted to control the lighting device alternately to generate a different lighting characteristic and to control the electronic image sensor (16) to detect the half-images with a frame rate of at least 50 Hz.

8. An imaging system in accordance with any one of the preceding claims, wherein the imaging system (10) has at least one playback system (50) for playing back the detected half-images that is electronically connected to an output of the taking system (14).

9. An imaging system in accordance with claim 8,
wherein the playback system (50) has a single monitor for presenting the detected half-images and a pair of eyepieces (54a, 54b), with the playback system (50) furthermore comprising a beam splitting device that directs the half-images detected in accordance with the one part optical path (28a) to the one eyepiece (54a) and the half-images detected in accordance with the other part optical path (28b) to the other eyepiece (54b); or
wherein the playback system (50) has two monitors (52a, 52b) for presenting the detected half-images, with an eyepiece being associated with each monitor (52a) and with the one monitor being provided for playing back the half-images detected in accordance with the one part optical path (28a) and the other monitor (52b) being provided for playing back the half-images detected in accordance with the other part optical path (28b).

10. An imaging system in accordance with claim 8 or claim 9,
wherein the imaging system (10) furthermore comprises an input device (86) that is adapted to fix a respective first image marking point and a respective second image marking point within the half-images played back at the playback system (50), with the respective first image marking point at the object (12) corresponding to a first object marking point (90) and the respective second image marking point at the object (12) corresponding to a second object marking point (92), said first and second object marking points being able to be arranged at different depths along the optical axis (A); and
wherein the imaging system (10) comprises an evaluation and control device (88) that is adapted to calculate a depth-dependent spacing (D) between the first object marking point (90) and the second object marking point (92).

11. An imaging system in accordance with any one of the preceding claims, wherein the imaging system (10) furthermore has at least one recording device (46) for recording the detected half-images that is electronically connected to an output of the image sensor (16) of the taking system (14).

12. An imaging system in accordance with any one of the preceding claims, wherein the imaging system (10) furthermore has at least one data transmission interface (48) for a telecommunication of the detected half-images that is electronically connected to an output of the image sensor (16) of the taking system (14).

13. An imaging system in accordance with any one of the preceding claims, wherein the taking system (14) has at least one first data output (40a) that is adapted to output the detected half-images to a playback system (50) for playing back the half-images; wherein the taking system (14) has at least one second data output (40b, 40c) that is adapted to output the detected half-images to a recording device (46) for recording the half-images or to output the detected half-images to a data network (48) for the telecommunication of the half-images; wherein the imaging system (10) furthermore comprises a lighting device for lighting the object in accordance with a variable lighting characteristic; wherein the synchronization and control device (38) is adapted to control the electronic image sensor (16) to detect the half-images at a frame rate of at least 50 Hz and to control the lighting device synchronously thereto to alternately generate at least one first and one second lighting characteristic; and wherein the synchronization and control device (38) is furthermore adapted to output at least one first stream of pairs of half-images at the first data output (40a) and at least one second stream of pairs of half-images at the second data output (40b, 40c), with the two streams of pairs of half-images differing at least partly from one another with respect to the respective lighting characteristic used for the generation of the half-images.

14. An imaging system in accordance with any one of the preceding claims, wherein the imaging system (10) furthermore has a selection device for selecting at least one of the named image groups that is adapted to control the imaging system (10) such that only the half-images associated with the selected image group are played back.

15. An imaging system in accordance with any one of the preceding claims, wherein the imaging system (10) has a position sensor (36) that is associated with the shutter assembly (32) and that is adapted to determine the angular position of the shutter assembly (32) and to transmit position signals corresponding to the determined angular position to the synchronization and control device (38), with the position signals comprising information on which of the part optical paths (28a, 28b) is instantaneously released or interrupted; and
wherein the synchronization and control device (38) is furthermore adapted to generate respective channel identifiers on the basis of the transmitted position signals and to link them to the half-images detected by the image sensor (16) such that each half-image is associated with the part optical path (28a, 28b) released for the detection of this half-image using the channel identifier linked hereto.

## Revendications

1. Système d'imagerie stéréoscopique sous la forme d'un microscope destiné à générer des paires de trames stéréoscopiques, comprenant un système de prise de vue (14), comportant :
- un unique capteur d'image électronique (16) qui comprend un obturateur électronique pour régler une période d'intégration de charge du capteur d'image (16);
- un objectif (18) destiné à générer une image d'un objet (12) sur le capteur d'image, l'objectif (18) comprenant un unique trajet optique d'imagerie d'axe optique central (A) ;
- un diaphragme d'ouverture double (24) agencé dans le trajet optique d'imagerie, qui comprend deux ouvertures (26a, 26b) écartées l'une de l'autre et décalées par rapport à l'axe optique (A), qui subdivisent le trajet optique d'imagerie en trajets optiques partiels respectifs (28a, 28b), les trajets optiques partiels (28a, 28b) se distinguant de par leur angle d'observation de l'objet (12) ;
- un diaphragme de fermeture (32) rotatif associé au diaphragme d'ouverture double (24), qui dégage en alternance temporelle selon une vitesse de rotation l'une des deux ouvertures (26a, 26b) pendant une période temporelle prédéterminée et qui referme l'autre des deux ouvertures (26a, 26b) au moins pendant cette période temporelle, afin de dégager en alternance temporelle l'un des deux trajets optiques partiels (28a, 28b) et d'interrompre l'autre des deux trajets optiques partiels (28a, 28b) ; et
- un dispositif de synchronisation et de commande (38) qui est adapté à synchroniser la période d'intégration de charge réglée par l'obturateur électronique du capteur d'image (16) et la vitesse de rotation du diaphragme de fermeture rotatif (32) l'une avec l'autre de telle sorte que le capteur d'image (16) saisit une trame stéréoscopique respective en se basant sur le trajet optique partiel (28a, 28b) dégagé, pendant ladite période temporelle ;
**caractérisé en ce que**
le système d'imagerie (10) est adapté à sélectionner l'une parmi au moins deux plages spectrales prédéterminées, qui est prise en compte par le capteur d'image (16) destiné à saisir une paire respective de trames stéréoscopiques, le système d'imagerie (10) étant en outre adapté à sélectionner la plage spectrale en alternance temporelle et à associer les paires de trames stéréoscopiques à une groupe d'images correspondant en fonction de la plage spectrale sélectionnée, de sorte que seulement celles des trames sont associées à chaque groupe d'images qui correspondent à la même plage spectrale sélectionnée.

2. Système d'imagerie selon la revendication 1,
dans lequel le dispositif de synchronisation et de commande (38) est adapté à adapter la vitesse de rotation du diaphragme de fermeture rotatif (32) à une fréquence d'images du capteur d'image (16), qui correspond à la période d'intégration de charge réglée ; ou
dans lequel le dispositif de synchronisation et de commande (38) est adapté à adapter une fréquence d'images du capteur d'image (16), qui correspond à la période d'intégration de charge réglée, à une vitesse de rotation prédéterminée du diaphragme de fermeture rotatif (32).

3. Système d'imagerie selon la revendication 1 ou 2,
le système d'imagerie (10) comprenant en outre un dispositif d'éclairage (56) qui est adapté à éclairer l'objet (12) en alternance depuis deux angles d'éclairage différents, à chaque angle d'éclairage étant associé l'un des trajets optiques partiels (28a, 29b), et le dispositif de synchronisation et de commande (38) est en outre adapté à activer l'angle d'éclairage respectif de façon synchrone au dégagement du trajet optique partiel (28a, 28b) associé.

4. Système d'imagerie selon la revendication 1 ou 2,
le système d'imagerie (10) comprenant en outre un dispositif d'éclairage (56) qui est adapté à générer au moins un faisceau d'éclairage (72) pour éclairer l'objet (12), au moins une lentille (20, 22) de l'objectif (18) comprenant au moins un évidement (76) par lequel la section transversale de ladite au moins une lentille (20, 22) se distingue de la forme d'une surface circulaire fermée (78), et ledit au moins un faisceau d'éclairage (72) s'étend le long de l'axe optique (A) à l'intérieur de l'évidement (76).

5. Système d'imagerie selon la revendication 4,
dans lequel ledit au moins un évidement (76), observé le long de l'axe optique (A), est décalé par rapport aux deux ouvertures (26a, 26b) du diaphragme d'ouverture double (24).

6. Système d'imagerie selon la revendication 4 ou 5,
dans lequel le dispositif d'éclairage (56) comprend au moins une lentille divergente (74) pour élargir ledit au moins un faisceau d'éclairage (72), ladite au moins une lentille divergente (74) étant agencée en arrière de ladite au moins une lentille (20, 22) par rapport à la direction de propagation dudit au moins un faisceau d'éclairage (72).

7. Système d'imagerie selon la revendication 1 ou 2,
le système d'imagerie (10) comprenant en outre un dispositif d'éclairage pour éclairer l'objet, le dispositif de synchronisation et de commande (38) étant adapté à piloter le dispositif d'éclairage en alternance pour générer des caractéristiques d'éclairage différentes et pour piloter le capteur d'image électronique (16) pour saisir les trames à une fréquence d'images d'au moins 50 Hz.

8. Système d'imagerie selon l'une des revendications précédentes,
le système d'imagerie (10) comprenant au moins un système de reproduction (50) pour reproduire les trames saisies, qui est connecté par voie électronique à une sortie du système de prise de vue (14).

9. Système d'imagerie selon la revendication 8,
dans lequel le système de reproduction (50) comprend un unique écran pour représenter les trames saisies et une paire d'oculaires (54a, 54b), le système de reproduction (50) comprenant en outre un dispositif diviseur de faisceau qui dirige vers l'un des oculaires (54a) les trames saisies selon l'un des trajets optiques partiels (28a) et qui dirige vers l'autre oculaire (54b) les trames saisies selon l'autre trajet optique partiel (28b) ; ou
dans lequel le système de reproduction (50) comprend deux écrans pour représenter les trames saisies, et un oculaire est associé à chaque écran (52a), l'un des écrans étant prévu pour reproduire les trames saisies selon l'un des trajets optiques partiels (28a), et l'autre écran (52b) étant prévu pour reproduire les trames saisies selon l'autre trajet optique partiel (28b).

10. Système d'imagerie selon la revendication 8 ou 9,
dans lequel le système d'imagerie (10) comprend en outre un dispositif d'entrée (86) qui est adapté à fixer un premier point de marquage d'image respectif et un second point de marquage d'image respectif à l'intérieur des trames reproduites sur le système de reproduction (50), le premier point de marquage d'image respectif sur l'objet (12) correspondant à un premier point de marquage d'objet (90), et le second point de marquage d'image respectif sur l'objet (12) correspondant au second point de marquage d'objet (92), qui peuvent être disposés à différentes profondeurs le long de l'axe optique (A), et
dans lequel le système d'imagerie (10) comprend un dispositif d'évaluation et de commande (88) qui est adapté à calculer une distance (D), dépendante de la profondeur, entre le premier point de marquage d'objet (90) et le second point de marquage d'objet (92).

11. Système d'imagerie selon l'une des revendications précédentes,
dans lequel le système d'imagerie (10) comprend au moins un dispositif d'enregistrement (46) pour enregistrer les trames saisies, qui est connecté par voie électronique à une sortie du capteur d'image (16) du système de prise de vue (14).

12. Système d'imagerie selon l'une des revendications précédentes,
dans lequel le système d'imagerie (10) comprend en outre au moins une interface de transmission de données (48) pour transmettre à distance les trames saisies, qui est connectée par voie électronique à une sortie du capteur d'image (16) du système de prise de vue (14).

13. Système d'imagerie selon l'une des revendications précédentes,
dans lequel le système de prise de vue (14) comprend en outre au moins une première sortie de données (40a) qui est adaptée à livrer les trames saisies à un système de reproduction (50) pour reproduire les trames, le système de prise de vue (14) comprenant au moins une seconde sortie de données (40b, 40c) qui est adaptée à livrer les trames saisies à un dispositif d'enregistrement (46) pour enregistrer les trames ou à livrer les trames saisies à un réseau de données (48) pour transmettre à distance les trames, le système d'imagerie (10) comprenant en outre un dispositif d'éclairage pour éclairer l'objet selon une caractéristique d'éclairage variable, le dispositif de synchronisation et de commande (38) étant adapté à piloter le capteur d'image électronique (16) pour saisir les trames à une fréquence d'images d'au moins 50 Hz et à piloter de façon synchrone le dispositif d'éclairage en alternance avec la génération d'au moins une première et une seconde caractéristique d'éclairage, et le dispositif de synchronisation et de commande (38) est en outre adapté à livrer à la première sortie de données (40a) au moins un premier flux de paires de trames et à livrer à une seconde sortie de données (40b, 40c) au moins un second flux de paires de trames, les deux flux de paires de trames se distinguant au moins partiellement l'un de l'autre par rapport à la caractéristique d'éclairage respectif utilisé pour générer les trames.

14. Système d'imagerie selon l'une des revendications précédentes,
dans lequel le système d'imagerie (10) comprend en outre un dispositif de sélection pour sélectionner au moins l'un desdits groupes d'images, qui est adapté à commander le système d'imagerie (10) de telle sorte que seules les trames associées au groupe d'images sélectionné sont reproduites.

15. Système d'imagerie selon l'une des revendications précédentes,
dans lequel le système d'imagerie (10) comprend un capteur de position (36) qui est associé au diaphragme de fermeture (32) et qui est adapté à déterminer la position angulaire du diaphragme de fermeture (32) et à transmettre au dispositif de synchronisation et de commande (38) des signaux de position correspondant à la position angulaire déterminée, les signaux de position comprenant des informations pour savoir quel des trajets optiques partiels (28a, 28b) est dégagé ou interrompu momentanément, et le dispositif de synchronisation et de commande (38) est en outre adapté à générer des identificateurs de canal respectifs en se basant sur les signaux de position transmis et à les chaîner avec les trames saisies par le capteur d'image (16) de telle sorte que chaque trame est associée au trajet optique partiel (28a, 28b), dégagé pour saisir de cette trame, en se basant sur l'identificateur de canal chaîné avec celle-ci.
